# EUROPEAN PATENT APPLICATION

(11) **EP 4 438 596 A1**
(43) Date of publication of application: **02.10.2024**
(21) Application number: 23305430.3
(22) Date of filing: 28.03.2023
(51) Int. Cl.: C07D 239/48, A61K 31/505, A61P 33/06

(54) **DIAMINOPYRIMIDINE DERIVATIVES AS HDAC AND DHFR DUAL-TARGETING INHIBITORS AND USE THEREOF IN PREVENTING AND TREATING MALARIA**

(71) Applicant: Université de Lille, 59800 Lille (FR); Institut Pasteur de Lille, 59800 Lille (FR); Centre Hospitalier Universitaire de Lille, 59000 Lille (FR); Institut National de la Santé et de la Recherche Médicale (INSERM), 75013 Paris (FR); Martin-Luther-Universität Halle-Wittenberg, 06108 Halle (Saale) (DE)
(72) Inventor: PIERROT, Christine, 59930 LA CHAPELLE D'ARMENTIERES (FR); KHALIFE, Jamal, 59130 LAMBERSART (FR); SIPPL, Wolfgang, 06114 HALLE (SAALE) (DE); GHAYZ, Ehab, 06122 HALLE (SAALE) (DE); ABDELSALAM, Mohamed, 06120 HALLE (SAALE) (DE)
(74) Representative: Regimbeau

(57) **Abstract**

The invention relates to a compound of formula (I): 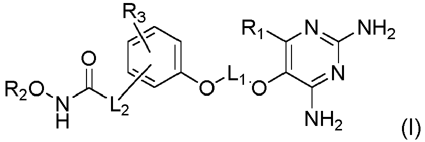 wherein
∘ L₁ is a (C₁-C₆) alkylene,
∘ R₁ is H, a (C₁-C₆)alkyl group or a (C₃-C₆)cycloalkyl group,
∘ R₂ is H, a (C₁-C₆)alkyl group, or a (C₁-C₆)alkylaryl group, said alkylaryl being optionally substituted by -OH, -OR^{a} or -OC(O)R^{b}, with R^{a} and R^{b} independently representing a (C₁-C₆)alkyl group,
∘ R₃ is H, a (C₁-C₆)alkyl group, a halogen, a (C₁-C₆)alkoxy group, -OH, -NHR^{c}, (C₁-C₆)haloalkyl group, -CONHR^{d}, -NHCOR^{e}, with R^{c} and R^{d} independently representing H or a (C₁-C₆)alkyl group, and R^{e} representing a (C₁-C₆)alkyl group,
∘ L₂ is a bond, a (C₁-C₁₂) alkylene, a (C₂-C₁₂) alkenylene, -NH-(C₁-C₁₂) alkylene, -NH-(C₂-C₁₂) alkenylene, -CO-NH- (C₁-C₁₂) alkylene, -CO-NH- (C₂-C₁₂) alkenylene, -O-(C₁-C₁₂) alkylene, -NH-CO-(C₁-C₁₂) alkylene or (C₁-C₁₂) alkylene-O-arylène-(C₂-C₁₂) alkenylene,
or a pharmaceutically acceptable salt or solvate thereof.

The compounds of formula (I) are useful as drugs, in particular for preventing or treating a parasitic disease, such as malaria.

## Description

### FIELD OF INVENTION

The present invention relates to dual targeting inhibitors of histone deacetylase (HDAC) and dihydrofolate reductase (DHFR), useful in the prevention and treatment of malaria.

### TEHCNICAL BACKGROUND

Malaria, caused by the apicomplexan parasites *Plasmodium* species, is among the most serious health problems worldwide. Malaria leads to an estimated 400,000 deaths per year mainly in children. The emergence of resistance against established drugs in *Plasmodium* populations emphasizes the need to pursue a better understanding of the biology of this parasite in order to identify and to evaluate novel targets and means for therapeutic benefits. Currently there is no licensed vaccine available for this parasite. Consequently, prevention and treatment rely heavily on drugs. Treatments of choice to ensure complete cure and to delay or avoid the emergence of resistant strains are artemisinin-based combination therapies (ACTs). In endemic countries, ACTs that use a double- or triple-combination are adapted to the transmission intensity, parasite resistance and uncomplicated versus severe malaria. The combination therapy of artemisinin and the dihydrofolate reductase (DHFR) inhibitor pyrimethamine (PYR, commonly used in the treatment of uncomplicated, chloroquine resistant, *P. falciparum* malaria, in combination with sulfadoxine) is still used against malaria even if PYR has to be used in high doses and PYR-resistant strains have been identified, especially in Africa.

Currently however, the use of ACTs and PYR is seriously questioned because of their reduced efficacy, as they can induce the emergence of resistant parasites and/or the early-stage parasites to enter a dormancy state and return to growth after drug removal.

Therefore, generating compounds with multi-target functionality could prevent rapid emergence of resistant strains and may provide promising new compounds for a more effective treatment of malaria through innovative combinations.

For instance, hybrid molecules derived from artemisinin and isoquinolines have recently been reported and shown to have the potential to enhance efficacy, improve safety, be cost-effective and reduce the propensity to elicit resistance relative to the parent drugs (Kouznetsov et al. Eur J Med Chem, 2009. 44(8): p. 3091-3113; Meunier Accounts of Chemical Research, 2008. 41(1): p. 69-77; Muregi et al. Drug Dev Res, 2010. 71(1): p. 20-32; Vandekerckhove et al. Bioorganic & Medicinal Chemistry, 2015. 23(16): p. 5098-5119;). However, these molecules were not further investigated.

*Plasmodium* parasites possess a substantial repertoire of enzymes with conserved functions, including those involved in chromatin remodeling and histone modifications which contribute to common histone marks and epigenetic mechanisms.

Histone modifying enzymes have been shown to be key in achieving spatio-temporal regulation of gene expression, and to play vital roles in the growth and survival of parasites. For instance, in *Plasmodium falciparum,* histone deacetylases (HDACs), histone acetyltransferases (HATs) and methyltransferases (HMTs) are involved in controlling cell cycle progression, particularly in controlling the variable surface gene expression related to the parasite's immune evasion. More importantly, compounds inhibiting histone modifying enzymes were shown to be able to disrupt parasite growth *in vitro.* The utility of HDACs as targets and their importance for parasite survival was confirmed by genetic and drug testing studies. Indeed, using random PiggyBac transposon mutagenesis in *P*. *falciparum,* three HDACs out of five seem to be essential.

However, most of the reported HDAC inhibitors (HDACi) have been shown to be active *in vitro* against *Plasmodium* falciparum, with no significant activity *in vivo* using rodent malaria model. Besides, the DHFR mentioned above is a key enzyme involved in the regeneration of folic acid. Its inhibition blocks the biosynthesis of purines and pyrimidines, which are essential for DNA synthesis and cell multiplication. This leads to failure of nuclear division at the time of schizont formation in erythrocytes and liver. Alongside PYR, compound P65 is also a potent DHFR inhibitor.

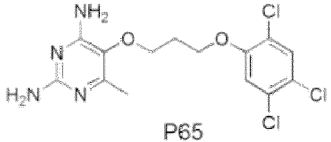

Hence, there is still a need for new dual-targeting compounds potent in the treatment and/or prevention of parasitic diseases such as malaria, which would allow to prevent or at least delay the emergence of parasitic resistance.

### SUMMARY OF THE INVENTION

The compounds of formula (I) as defined below are bi-functional in that they contain:
- a HDAC inhibitor scaffold: the benzhydroxamic acid moiety of the compounds of formula (I), and
- a DHFR-targeting group, previously reported to be active against PYR-resistant *P*. *falciparum* strains (the 2,4-diamino-pyrimidin-5-ol moiety also present in P65).

All tested compounds showed indeed an inhibitory *in vitro* activity against PfDFHR, and are active against P. *falciparum* strains (in particular 3D7 strains and multidrug-resistant line Dd2) at nanomolar levels, with selectivity indices (ratio of the minimum inhibitory concentration of HEK293 cells (50 µM) to the IC₅₀ of Pf3D7) greater than 1.10⁶.

Therefore, in a first aspect, the invention concerns a compound of formula (I) below: 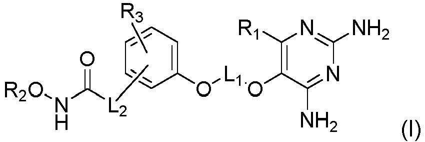 wherein
∘ L₁ is a (C₁-C₆) alkylene,
∘ R₁ is H, a (C₁-C₆)alkyl group or a (C₃-C₆)cycloalkyl group,
∘ R₂ is H, a (C₁-C₆)alkyl group, or a (C₁-C₆)alkylaryl group, said alkylaryl being optionally substituted by -OH, -OR^{a} or -OC(O)R^{b}, with R^{a} and R^{b} independently representing a (C₁-C₆)alkyl group,
∘ R₃ is H, a (C₁-C₆)alkyl group, a halogen, a (C₁-C₆)alkoxy group, -OH, -NHR^{c}, (C₁-C₆)haloalkyl group, -CONHR^{d}, -NHCOR^{e}, with R^{c} and R^{d} independently representing H or a (C₁-C₆)alkyl group, and R^{e} representing a (C₁-C₆)alkyl group,
∘ L₂ is a bond, a (C₁-C₁₂) alkylene, a (C₂-C₁₂) alkenylene, -NH-(C₁-C₁₂) alkylene, -NH-(C₂-C₁₂) alkenylene, -CO-NH- (C₁-C₁₂) alkylene, -CO-NH- (C₂-C₁₂) alkenylene, -O-(C₁-C₁₂) alkylene, -NH-CO-(C₁-C₁₂) alkylene or (C₁-C₁₂) alkylene-O-arylene-(C₂-C₁₂) alkenylene,
or a pharmaceutically acceptable salt or solvate thereof.

In another aspect, the present invention relates to a pharmaceutical or veterinary composition comprising a compound of formula (I) as described above or below, and a pharmaceutically or veterinary acceptable carrier.

In another aspect, the invention relates to a compound of formula (I) as defined above and below for use as a drug, in particular for preventing or treating a parasitic disease such as malaria.

In another aspect, the present invention relates to a kit comprising:
- the composition of the invention, and
- a second composition comprising at least another therapeutic compound,
as a combination product for simultaneous, separate and staggered use for preventing or treating a parasitic disease such as malaria.

In another aspect, the present invention encompasses methods for preparing the compounds of formula (I) as defined above. An exemplary method comprises the following steps:
a) Subjecting a compound of formula (II) below: 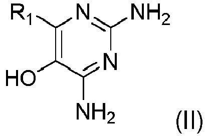
   with R₁ as defined above in connection with the compounds of formula (I),
   to a Williamson ether synthesis with a compound of formula (III) below: 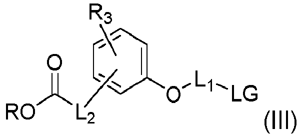
   with R₃, L₁ and L₂ as defined above in connection with the compounds of formula (I),
   R representing a (C₁-C₄)alkyl group, preferably a methyl or ethyl group, more preferably a methyl group, and
   LG representing a leaving group,
   to obtain a compound of formula (IV) below 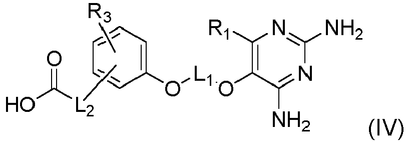
   with R₁, R₃,L₁ and L₂ as defined above in connection with the compounds of formula (I), and
b) Subjecting the compound of formula (IV) as obtained in step a) to a peptidic coupling with a compound formula (V) below:

   H₂N-O-PG (V)

   PG being a tetrahydropyranyl protecting group or a (C₁-C₆)alkyl group, or a (C₁-C₆)alkylaryl group, said alkylaryl being optionally substituted by -OH, -OR^{a} or-OC(O)R^{b}, with R^{a} and R^{b} independently representing a (C₁-C₆)alkyl group,
or an acid addition salt of the compound formula (V), such as a hydrochloride salt thereof.

### FIGURES

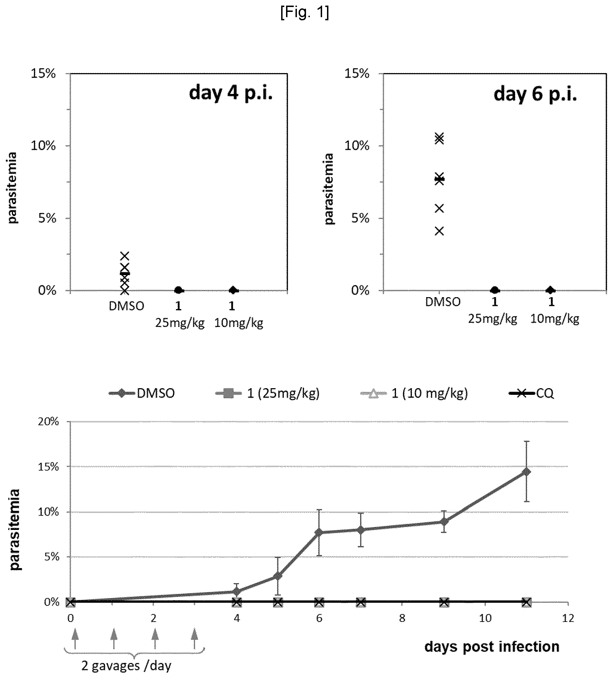

[Fig. 1] Experiment 1. **1.HCl** dual-targeting compound is active *in vivo* against *Plasmodium berghei.* Balb/C mice were infected with *P*. *berghei* (10⁶ iRBC, ip route) and treated orally with **1.HCl** (10mg/kg and 25mg/kg) or vehicle twice a day on days 0,1,2,3 post infection. Infected control mice received DMSO (7%, same formulation as for **1.HCl**) following the same protocol, and three mice received 4 doses of chloroquine (**CQ**, 10mg/kg, *p.o*.), as positive control. Parasitemia was followed on blood smears.

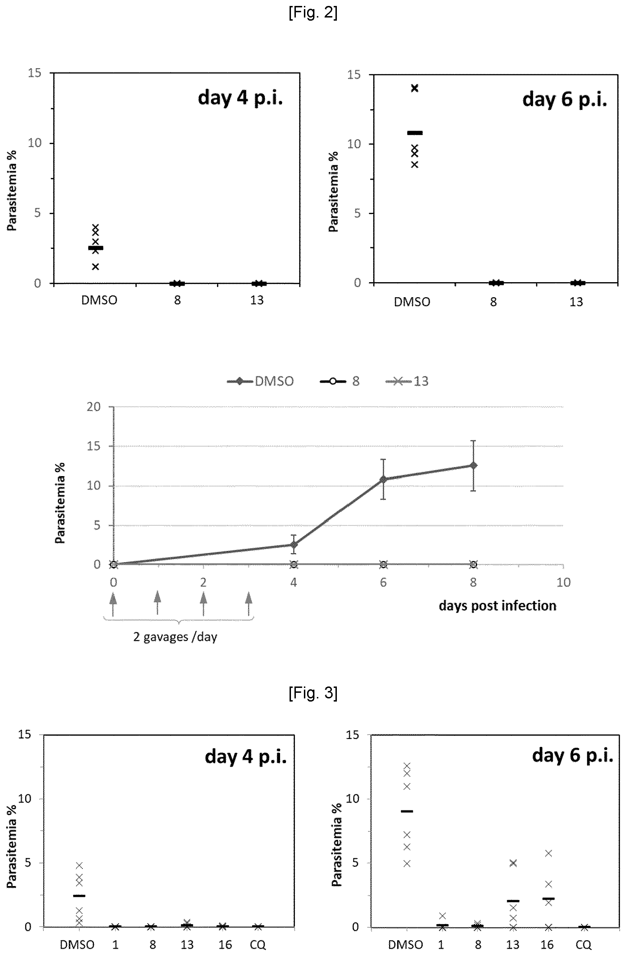

[Fig. 2] Experiment 2. **8.HCl** and **13.HCl** dual-targeting compounds are active *in vivo* against *Plasmodium berghei.* Balb/C mice were infected with *P*. *berghei* (10⁶ iRBC, ip route) and treated orally with **8.HCl** or **13.HCl** (10mg/kg) or vehicle twice a day on days 0,1,2,3 post infection. Infected control mice received DMSO (7%, same formulation as for **8.HCl** and **13.HCl**) following the same protocol. Parasitemia was followed on blood smears.
[Fig. 3] Experiment 3. Compared protection of *P*. *berghei*-infected mice treated with 1mg/kg **1.HCl**, **8.HCl**, **13.HCl** or **16** dual-targeting compounds. Balb/C mice were infected with *P*. *berghei* (day 0, 10⁶ iRBC, ip route) and treated orally with **1.HCl**, **8.HCl**, **13.HCl** or **16** (1mg/kg) or vehicle twice a day on days 0,1,2,3 post infection. Infected control mice received DMSO (6%, same formulation as for **1.HCl**, **8.HCl**, **13.HCl** and **16**) following the same protocol, and three mice received 4 doses of chloroquine (**CQ**, 10mg/kg, *p.o*.), as positive control. Parasitemia was followed on blood smears.

### DEFINITIONS

The term "halogen", as used in the present invention, refers to a fluorine, bromine, chlorine or iodine atom, preferably a chlorine or fluorine atom.

The term "(C₁-C₆)alkyl", as used herein, refers to a linear or branched saturated hydrocarbon chain containing from 1 to 6 carbon atoms including, but not limited to, methyl, ethyl, n-propyl, iso-propyl, n-butyl, iso-butyl, sec-butyl, t-butyl, n-pentyl, n-hexyl, and the like.

The term "(C₂-C₆)alkenyl", as used herein, refers to a linear or branched unsaturated hydrocarbon chain containing from 2 to 6 carbon atoms and comprising at least one double bond including, but not limited to, ethenyl, propenyl, butenyl, pentenyl, hexenyl and the like. The term "(C₃-C₁₀)cycloalkyl", as used herein, refers to a hydrocarbon monocyclic or bicyclic (fused) ring having 3 to 10 carbon atoms including, but not limited to, cyclopropyl, cyclopentyl, cyclohexyl and the like.

The term "(C₁-C₆)haloalkyl", as used herein, refers to a (C₁-C₆)alkyl group as defined herein substituted by at least one halogen atom, and preferably by at least one fluorine atom. The (C₁-C₆)haloalkyl is preferably a (C₁-C₆)fluoroalkyl. It is in particular a trifluoromethyl group (CF₃).

The term "(C₁-C₆)alkoxy", as used herein, refers to a (C₁-C₆)alkyl group as defined above bound to the molecule via an oxygen atom, including, but not limited to, methoxy, ethoxy, n-propoxy, iso-propoxy, n-butoxy, iso-butoxy, sec-butoxy, t-butoxy, n-pentoxy, n-hexoxy, and the like.

The term "aryl group" as used herein alone or as part of another group denotes homocyclic aromatic groups, preferably monocyclic or bicyclic (fused) groups, containing from 6 to 12 carbons in the ring portion, such as phenyl, naphthyl and indenyl. Phenyl is the most preferred aromatic group.

As used herein, the term "alkylaryl" refers to an aryl group as defined above bound to the molecule via an alkyl group as defined above; in other words, it is a (C₁-C₆)alkyl-aryl group. Preferably, the alkylaryl group is a benzyl group.

The term "C₁-C₁₂ alkylene" as used herein, refers to a linear or branched divalent saturated hydrocarbon chain containing from 1 to 12 carbon atoms - preferably from 1 to 6 carbon atoms - including, but not limited to, C₁-alkylene, *i.e.* a methylene of formula -CH₂-, a C₂ alkylene, a C₃ alkylene, a C₄ alkylene, a C₅ alkylene and a C₆ alkylene.

The term "C₂-C₁₂ alkenylene", as used in the present invention, refers to a linear or branched divalent unsaturated hydrocarbon chain containing from 2 to 12 carbon atoms - preferably from 2 to 6 carbon atoms - and comprising at least one double bond including, but not limited to, ethenylene, propenylene, butenylene, pentenylene, hexenylene and the like. An alkenylene preferably contains non triple bond, and advantageously only one double bond.

The term "C₁-C₁₂ arylene" as used herein, refers to a divalent homocyclic aromatic group, preferably monocyclic or bicyclic (fused) group, containing from 6 to 12 carbons in the ring portion, such as phenylene, naphthylene and indenylene. Phenylene, especially -1,3-phenylene-, is the most preferred arylene group.

The term "leaving group" as used in the present invention refers to a chemical group which can be easily replaced with a nucleophile during a nucleophile substitution reaction, the nucleophile being in the present case an alcohol, generally a compound of formula (II) as defined herein. Such a leaving group can be in particular a halogen atom or a sulfonate, preferably a bromine atom. The sulfonate is in particular a group -OSO₂-R' with R' representing a (C₁-C₆) alkyl, aryl, aryl-(C₁-C₆)-alkyl or (C₁-C₆)-alkyl-aryl group. The sulfonate can be in particular a mesylate (CH₃-S(O₂)O-), a triflate (CF₃-S(O)₂O-) or a tosylate (p-Me-C₆H₄-S(O)₂O-).

As used herein, a "THP protecting group" is a tetrahydropyranyl group of formula

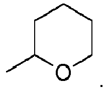

Also, in the present invention, Me stands for methyl, Ph stands for phenyl, Bn stands for benzyl and Bz stands for benzoyl, Ac stands for acetyl. More generally, the abbreviations used to refer to chemical groups have the meaning commonly known in the art.

### DETAILED DESCRIPTION

### Compounds of formula (I)

The invention thus concerns a compound of formula (I) below: 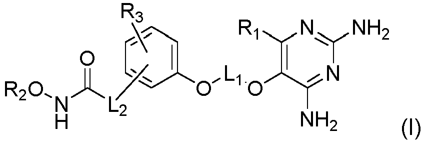 wherein
∘ L₁ is a (C₂-C₆) alkylene,
∘ R₁ is H, a (C₁-C₆)alkyl group or a (C₃-C₆)cycloalkyl group,
∘ R₂ is H, a (C₁-C₆)alkyl group, or a (C₁-C₆)alkylaryl group, said alkylaryl being optionally substituted by -OH, -OR^{a}, -OC(O)R^{b}, with R^{a} and R^{b} independently representing a (C₁-C₆)alkyl group (preferably a (C₁-C₄)alkyl group),
∘ R₃ is H, a (C₁-C₆)alkyl group, a halogen, a (C₁-C₆)alkoxy group, -OH, -NHR^{c}, (C₁-C₆)haloalkyl group, -CONHR^{d}, -NHCOR^{e}, with R^{c} and R^{d} independently representing H or a (C₁-C₆)alkyl group, and R^{e} representing a (C₁-C₆)alkyl group,
∘ L₂ is a bond, a (C₁-C₁₂) alkylene, a (C₂-C₁₂) alkenylene, -NH-(C₁-C₁₂) alkylene, -NH-(C₂-C₁₂) alkenylene, -CO-NH- (C₁-C₁₂) alkylene, -CO-NH- (C₂-C₁₂) alkenylene, -O-(C₁-C₁₂) alkylene, -NH-CO-(C₁-C₁₂) alkylene or (C₁-C₁₂) alkylene-O-arylene-(C₂-C₁₂) alkenylene,
or a pharmaceutically acceptable salt or solvate thereof.

In some embodiments, L₁ is a linear (C₂-C₆) alkylene. Preferably, L₁ is a (C₂-C₄) alkylene, such as -CH₂-CH₂- or -CH₂-CH₂-CH₂-, more preferably -CH₂-CH₂-CH₂-.

In some embodiments, R₁ is H, methyl, ethyl, n-propyl, cyclopropyl, n-butyl or isobutyl. In some embodiments, R₁ is H or a C₁-C₃ alkyl or a cyclopropryl group, preferably ethyl.

In some embodiments, R₂ is H, a (C₁-C₄)alkyl group, or a (C₁-C₄)alkylphenyl group (preferably benzyl), said phenyl being optionally substituted by -OMe, -OEt, -OAc, advantageously at the para-position. Typically, R₂ is H, benzyl or *p*-AcO-Ph-CH₂-.

In some embodiments, R₃ is H, a (C₁-C₄)alkyl group, a halogen, a (C₁-C₃)alkoxy group, -OH, - NH₂, a (C₁-C₃)fluoroalkyl group (preferably a -CF₃), -CONH₂, -NHCOCH₃. Preferably, R₃ is H, halogen or a methoxy group, more preferably H.

In some embodiments, L₂ is in the ortho position, so that the compound of formula (I) is best represented by the general formula (I'): 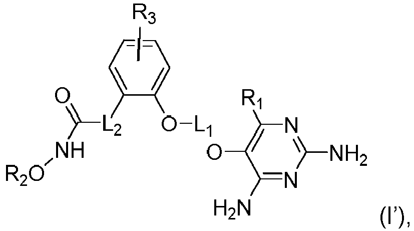 with L₁, L₂, R₁, R₂ and R₃ as defined herein.

In some embodiments, L₂ is a bond, a (C₁-C₆)alkylene, a (C₂-C₆)alkenylene, -NH-(C₁-C₆) alkylene, -NH-(C₂-C₆)alkenylene, -CO-NH-(C₁-C₆)alkylene, -CO-NH-(C₂-C₆)alkenylene, -O-(C₁-C₆) alkylene, -NH-CO-(C₁-C₆) alkylene or (C₁-C₆)alkylene-O-arylene-(C₂-C₆) alkenylene. In some embodiments, L₂ is a bond, a (C₁-C₁₂) alkylene or a (C₂-C₁₂) alkenylene, -CO-NH- (C₁-C₁₂) alkylene, -CO-NH- (C₂-C₁₂) alkenylene, or (C₁-C₁₂) alkylene-O-arylene-(C₂-C₁₂) alkenylene, preferably a bond, a (C₁-C₆) alkylene or a (C₂-C₆) alkenylene, -CO-NH- (C₁-C₆) alkylene, -CO-NH- (C₂-C₆) alkenylene, or (C₁-C₆) alkylene-O-arylene-(C₂-C₆) alkenylene. More preferably, L₂ is a bond, -CH₂-CH₂-, -CH=CH-, -CH₂-O-Ph-CH=CH-, -CO-NH-(CH₂)₆-, - NH-CO-(CH₂)₆-, -NH-CO-(CH₂)₄-.

In some particular embodiments:
∘ L₁ is a (C₂-C₄) alkylene,
∘ R₁ is H, a (C₁-C₄)alkyl group or a (C₃-C₄)cycloalkyl group, preferably R₁ is H or a C₁-C₃ alkyl or a cyclopropryl group
∘ R₂ is H, or a (C₁-C₄)alkylphenyl group, said phenyl being optionally substituted on the aryl moiety by -OR^{a} or -OC(O)R^{b}, with R^{a} and R^{b} independently representing a (C₁-C₄)alkyl group, preferably H, benzyl or *p*-AcO-Ph-CH₂-.
∘ R₃ is H, a (C₁-C₄)alkyl group, a halogen, a (C₁-C₄)alkoxy group, -OH, -NH₂, -CF₃, - CONH₂, -NHCOCH₃, preferably H, halogen or a methoxy group;
∘ L₂ is a bond, a (C₁-C₁₂) alkylene or a (C₂-C₁₂) alkenylene, -CO-NH- (C₁-C₁₂) alkylene, - CO-NH- (C₂-C₁₂) alkenylene, -NH-CO-(C₁-C₆) alkylene or (C₁-C₁₂) alkylene-O-arylene-(C₂-C₁₂) alkenylene, preferably a bond, -CH₂-CH₂- or -CH=CH-,-CHz-O-Ph-CH=CH-, - CO-NH-(CH₂)₆-, -NH-CO-(CH₂)₆-, -NH-CO-(CH₂)₄-.
or a pharmaceutically acceptable salt or solvate thereof.

For the purpose of the invention, the term "pharmaceutically acceptable" is intended to mean what is useful to the preparation of a pharmaceutical composition, and what is generally safe and non-toxic, for a pharmaceutical use.

Typically, the compounds of the present invention may be in the form of free bases or pharmaceutically acceptable acid addition salts thereof. The term "pharmaceutically acceptable salts" are salts commonly used to form alkali metal salts and to form addition salts of free acids or free bases. The nature of the salt may vary, provided that it is pharmaceutically acceptable. Suitable pharmaceutically acceptable acid addition salts of compounds for use in the present methods may be prepared from an inorganic acid or from an organic acid. Examples of such inorganic acids are hydrochloric, hydrobromic, hydroiodic, nitric, carbonic, sulfuric and phosphoric acid. Appropriate organic acids may be selected from formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, 4-hydroxybenzoic, phenylacetic, mandelic, embonic (pamoic), methanesulfonic, ethanesulfonic, benzenesulfonic, pantothenic, 2-hydroxyethanesulfonic, toluenesulfonic, sulfanilic, cyclohexylaminosulfonic, stearic, algenic, hydroxybutyric, salicylic, galactaric and galacturonic acid. All of these salts may be prepared by conventional means from the corresponding compound by reacting, for example, the appropriate acid with any of the compounds of the invention.

In particular embodiments, the compounds of formula (I) are in the form of an HCl addition salt.

Advantageously, the compound of formula (I) is selected from the group consisting of:

| | |
|---|---|
| **1** | 3-{2-[3-(2,4-Diamino-6-ethylpyrimidin-5-yloxy)propoxy]phenyl}-N-hydroxypropanamide, |
| **2** | 3-{2-[2-(2,4-Diamino-6-ethylpyrimidin-5-yloxy)ethoxy]phenyl}-N-hydroxypropanamide, |
| **3** | 3-{2-[4-(2,4-Diamino-6-ethylpyrimidin-5-yloxy)butoxy]phenyl}-N-hydroxypropanamide, |
| **4** | 3-{2-[3-(2,4-Diamino-6-ethylpyrimidin-5-yloxy)propoxy]-4-methoxyphenyl}-N-hydroxypropanamide |
| **5** | 3-{4-Chloro-2-[3-(2,4-diamino-6-ethylpyrimidin-5-yloxy)propoxy]phenyl}-*N-*hydroxypropanamide |
| **6** | *N*-(4-Acetoxybenzyloxy)-3-(2-(3-(2,4-diamino-6-ethylpyrimidin-5-yloxy)propoxy)phenyl)propanamide |
| **7** | *N*-(Benzyloxy)-3-(2-(3-(2,4-diamino-6-ethylpyrimidin-5-yloxy)propoxy)phenyl)propanamide |
| **8** | 3-[3-(2,4-Diamino-6-ethylpyrimidin-5-yloxy)propoxy]-*N*-hydroxybenzamide |
| **9** | 4-Chloro-3-[3-(2,4-diamino-6-ethylpyrimidin-5-yloxy)propoxy]-*N*-hydroxybenzamide |
| **10** | 3-(3-[2,4-Diamino-6-ethylpyrimidin-5-yloxy)propoxy]-4-fluoro-*N*-hydroxybenzamide |
| **11** | *N*-(4-Acetoxybenzyloxy)-3-[3-(2,4-diamino-6-ethylpyrimidin-5-yloxy)propoxy]benzamide |
| **12** | *N*-(Benzyloxy)-3-[3-(2,4-diamino-6-ethylpyrimidin-5-yloxy)propoxy]benzamide |
| **13** | 3-{2-[3-(2,4-Diamino-6-ethylpyrimidin-5-yloxy)propoxy]phenyl}-*N*-hydroxyacrylamide |
| **14** | 3-{2-[3-(2,4-Diamino-6-ethylpyrimidin-5-yloxy)propoxy]-4-methoxyphenyl}-*N-*hydroxyacrylamide |
| **15** | 3-{4-Chloro-2-[3-(2,4-diamino-6-ethylpyrimidin-5-yloxy)propoxy]phenyl}-*N-*hydroxyacrylamide |
| **16** | *N*-(4-Acetoxybenzyloxy)-3-{2-[3-(2,4-diamino-6-ethylpyrimidin-5-yloxy)propoxy]phenyl}acrylamide |
| **17** | *N*-(Benzyloxy)-3-{2-[3-(2,4-diamino-6-ethylpyrimidin-5-yloxy)propoxy]phenyl}acrylamide |
| **18** | 3-{3-[2-(2,4-Diamino-6-ethylpyrimidin-5-yloxy)ethoxy]phenyl}-*N*-hydroxyacrylamide |
| **19** | 3-{3-[3-(2,4-Diamino-6-ethylpyrimidin-5-yloxy)propoxy]phenyll-*N*-hydroxyacrylamide |
| **20** | 3-{4-[3-(2,4-Diamino-6-ethylpyrimidin-5-yloxy)propoxy]phenyl}-*N*-hydroxyacrylamide |
| **21** | 3-(3-{3-[3-(2,4-Diamino-6-ethylpyrimidin-5-yloxy)propoxy]benzyloxy}phenyl)-*N-*hydroxyacrylamide |
| **22** | 4-(2-{3-[(2,4-Diamino-6-ethylpyrimidin-5-yl)oxy]propoxy}phenyl)-*N*-hydroxybutanamide |
| **23** | 5-(2-{3-[(2,4-Diamino-6-ethylpyrimidin-5-yl)oxy]propoxy}phenyl)-*N-*hydroxypentanamide |
| **24** | 3-(2-{3-[(2,4-Diamino-6-cyclopropylpyrimidin-5-yl)oxy]propoxy}phenyl)-*N-*hydroxypropanamide |
| **25** | 3-(3-{3-[(2,4-Diamino-6-ethylpyrimidin-5-yl)oxy]propoxy}phenyl)-*N-*hydroxypropanamide |
| **26** | 3-(4-{3-[(2,4-Diamino-6-ethylpyrimidin-5-yl)oxy]propoxy}phenyl)-*N-*hydroxypropanamide |
| **27** | 4-{3-[(2,4-Diamino-6-ethylpyrimidin-5-yl)oxy]propoxy}-*N*-hydroxybenzamide |
| **28** | *N*¹-(2-{3-[(2,4-diamino-6-ethylpyrimidin-5-yl)oxy]propoxy}phenyl)-*N*⁶-hydroxyadipamide |
| **29** | *N*¹-(2-(3-((2,4-diamino-6-ethylpyrimidin-5-yl)oxy)propoxy)phenyl)-*N*⁸-hydroxyoctanediamide |
| **30** | 3-{3-[(2,4-Diamino-6-ethylpyrimidin-5-yl)oxy]propoxy}-*N*-[7-(hydroxyamino)-7-oxoheptyl]benzamide, and |
| **31** | 4-{3-[(2,4-Diamino-6-ethylpyrimidin-5-yl)oxy]propoxy}-*N*-[7-(hydroxyamino)-7-oxoheptyl]benzamide, |

or a pharmaceutically acceptable solvate or a salt thereof, in particular a hydrochloride salt thereof.

Preferably, the compound is selected from the group consisting of:

| | |
|---|---|
| **1** | 3-{2-[3-(2,4-Diamino-6-ethylpyrimidin-5-yloxy)propoxy]phenyl}-*N*-hydroxypropanamide |
| **2** | 3-{2-[2-(2,4-Diamino-6-ethylpyrimidin-5-yloxy)ethoxy]phenyl}-*N*-hydroxypropanamide |
| **3** | 3-{2-[4-(2,4-Diamino-6-ethylpyrimidin-5-yloxy)butoxy]phenyl}-*N*-hydroxypropanamide |
| **4** | 3-{2-[3-(2,4-Diamino-6-ethylpyrimidin-5-yloxy)propoxy]-4-methoxyphenyl}-*N-*hydroxypropanamide |
| **5** | 3-{4-Chloro-2-[3-(2,4-diamino-6-ethylpyrimidin-5-yloxy)propoxy]phenyl}-*N-*hydroxypropanamide |
| **6** | *N*-(4-Acetoxybenzyloxy)-3-(2-(3-(2,4-diamino-6-ethylpyrimidin-5-yloxy)propoxy)phenyl)propanamide |
| **7** | *N*-(Benzyloxy)-3-(2-(3-(2,4-diamino-6-ethylpyrimidin-5-yloxy)propoxy)phenyl)propanamide |
| **8** | 3-[3-(2,4-Diamino-6-ethylpyrimidin-5-yloxy)propoxy]-*N*-hydroxybenzamide |
| **9** | 4-Chloro-3-[3-(2,4-diamino-6-ethylpyrimidin-5-yloxy)propoxy]-*N*-hydroxybenzamide |
| **10** | 3-(3-[2,4-Diamino-6-ethylpyrimidin-5-yloxy)propoxy]-4-fluoro-*N*-hydroxybenzamide |
| **11** | *N*-(4-Acetoxybenzyloxy)-3-[3-(2,4-diamino-6-ethylpyrimidin-5-yloxy)propoxy]benzamide |
| **12** | *N*-(Benzyloxy)-3-[3-(2,4-diamino-6-ethylpyrimidin-5-yloxy)propoxy]benzamide |
| **13** | 3-{2-[3-(2,4-Diamino-6-ethylpyrimidin-5-yloxy)propoxy]phenyl}-*N*-hydroxyacrylamide |
| **14** | 3-{2-[3-(2,4-Diamino-6-ethylpyrimidin-5-yloxy)propoxy]-4-methoxyphenyl}-*N-*hydroxyacrylamide |
| **15** | 3-{4-Chloro-2-[3-(2,4-diamino-6-ethylpyrimidin-5-yloxy)propoxy]phenyl}-*N-*hydroxyacrylamide |
| **16** | *N*-(4-Acetoxybenzyloxy)-3-{2-[3-(2,4-diamino-6-ethylpyrimidin-5-yloxy)propoxy]phenyl}acrylamide |
| **17** | *N*-(Benzyloxy)-3-{2-[3-(2,4-diamino-6-ethylpyrimidin-5-yloxy)propoxy]phenyl}acrylamide |
| **18** | 3-{3-[2-(2,4-Diamino-6-ethylpyrimidin-5-yloxy)ethoxy]phenyl}-*N*-hydroxyacrylamide |
| **19** | 3-{3-[3-(2,4-Diamino-6-ethylpyrimidin-5-yloxy)propoxy]phenyl}-*N*-hydroxyacrylamide |
| **20** | 3-{4-[3-(2,4-Diamino-6-ethylpyrimidin-5-yloxy)propoxy]phenyl}-*N*-hydroxyacrylamide |
| **21** | 3-(3-{3-[3-(2,4-Diamino-6-ethylpyrimidin-5-yloxy)propoxy]benzyloxy}phenyl)-*N-*hydroxyacrylamide |
| **30** | 3-{3-[(2,4-Diamino-6-ethylpyrimidin-5-yl)oxy]propoxy}-*N*-[7-(hydroxyamino)-7-oxoheptyl]benzamide,and |
| **31** | 4-{3-[(2,4-Diamino-6-ethylpyrimidin-5-yl)oxy]propoxy}-*N*-[7-(hydroxyamino)-7-oxoheptyl]benzamide |

or a pharmaceutically acceptable solvate or a salt thereof, in particular a hydrochloride salt thereof.

Even more preferably, the compound of formula (I) is: 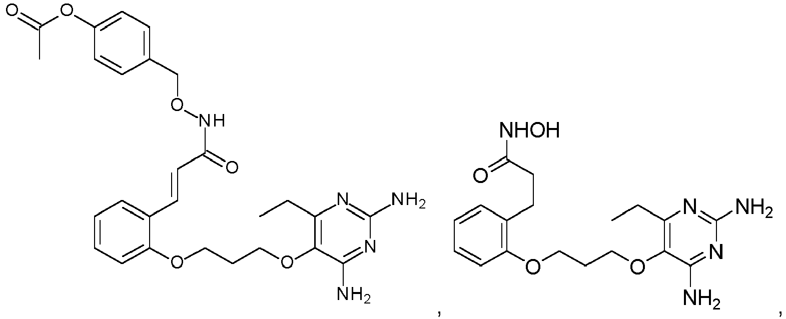 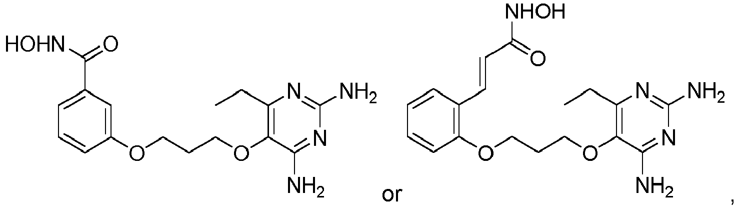 or a pharmaceutically acceptable solvate or a salt thereof, in particular a hydrochloride salt thereof.

The compounds of formula (I) as described above may exist in tautomeric, diastereomeric or enantiomeric forms. The present invention contemplates all such compounds, including cis- and trans-diastereomers, E- and Z-stereomers, R- and S-enantiomers, diastereomers, d-isomers, I-isomers, the racemic mixtures thereof and other mixtures thereof. Pharmaceutically acceptable salts of such tautomeric, diastereomeric or enantiomeric forms are also included within the invention. The terms "cis" and "trans", as used herein, denote a form of geometric isomerism in which two carbon atoms connected by a double bond will each have a hydrogen atom on the same side of the double bond ("cis") or on opposite sides of the double bond ("trans").

### Process for preparing the compounds of formula (I)

The compounds of formula (I) may be prepared by a process comprising the following steps:
a) Subjecting a compound of formula (II) below: 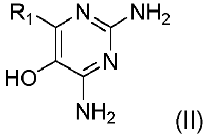
   with R₁ as defined above in connection with the compounds of formula (I),
   to a Williamson ether synthesis with a compound of formula (III) below: 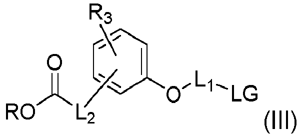
   with R₃, L₁ and L₂ as defined above in connection with the compounds of formula (I),
   R representing a (C₁-C₄)alkyl group, preferably a methyl or ethyl group, more preferably a methyl group, and
   LG representing a leaving group,
   to obtain a compound of formula (IV) below 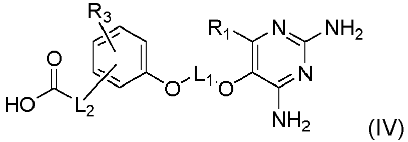
   with R₁, R₃,L₁ and L₂ as defined above in connection with the compounds of formula (I), and
b) Subjecting the compound of formula (IV) as obtained in step a) to a peptidic coupling with a compound formula (V) below:

   H₂N-O-PG (V)

   PG being a THP protecting group or a (C₁-C₆)alkyl group, or a (C₁-C₆)alkylaryl group, said alkylaryl being optionally substituted by -OH, -OR^{a}, -OC(O)R^{b}, with R^{a} and R^{b} independently representing a (C₁-C₆)alkyl group,
or an acid addition salt of the compound formula (V), such as a hydrochloride salt thereof.

LG is preferably a halogen, even more preferably a bromine.

When PG is a THP protecting group, the process further comprises a step c) of deprotecting the THP protecting group, typically under acidic conditions such as by contacting the compound of formula (IV) with aqueous HCl in a polar organic solvent, such as tetrahydrofuran (THF).

When the process is carried out to prepare a compound of formula (I), wherein R₂ is H and L₂ is a (C₂-C₁₂) alkenylene, PG in the compound of formula (V) is a THP protecting group. The process then further comprises a step c) of deprotecting the THP protecting group (typically under acidic conditions), thereby yielding the compound of formula (I).

When the process is carried out to prepare a compound of formula (I), wherein R₂ is H and L₂ is a bond or a (C₁-C₁₂) alkylene, PG in the compound of formula (V) is typically a benzyl group, and the process further comprises a step c) of deprotecting the PG group by hydrogenation, typically using Pd/C under a H₂ atmosphere, thereby yielding the compound of formula (I).

The Williamson ether synthesis a) is typically carried out using a base in a protic solvent. The base is preferably a mineral base, such as a hydroxide salt, advantageously NaOH or LiOH. The solvent is preferably dimethylformamide (DMF). The Williamson ether synthesis a) is usually performed at room temperature.

Of note, step a) comprises a concomitant saponification step.

The peptidic coupling of step b) is typically carried out using an acid activating agent such as Benzotriazole-1-yl-oxytripyrrolidinophosphonium hexafluorophosphate (PyBOP) or 2-(7-Aza-1H-benzotriazole-1-yl)-1,1,3,3-tetramethyluronium hexafluorophosphate (HATU). The peptidic coupling of step b) is further typically carried out in the presence a base, preferably an organic base, such as an amine of formula (VI) NR_{f}R_{g}Rₕ, with R_{f}, R_{g} and Rₕ independently representing a (C₁-C₆)alkyl group. Preferably R_{f}, R_{g} and Rₕ are independently representing a (C₁-C₃)alkyl group. The preferred base is diisopropylethylamine (DIPEA).

The compound of formula (III) may be prepared through a Mitsunobu reaction of a compound for formula (VII) below: 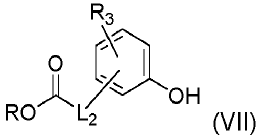
with L₂ and R₃ as defined above in connection with the compounds of formula (I), and
R representing a (C₁-C₄)alkyl group, preferably a methyl or ethyl group, more preferably a methyl group, with a compound of formula (VIII):

   HO-L₁-LG (VIII)
with L₁ as defined above in connection with the compounds of formula (I), and LG representing a leaving group.

The Mitsunobu reaction is typically carried out in the presence of a triarylphosphine of formula PAr₁Ar₂Ar₃, with Ar₁, Ar₂ and Ar₃ independently representing an aryl group, preferably a phenyl group. The preferred triarylphosphine is triphenylphosphine.

The Mitsunobu reaction is also typically carried out in the presence of a dialkyl azodicarboxylate of formula RᵢO(O)C-N=N-C(O)ORⱼ, with Rᵢ and Rⱼ independently representing a (C₁-C₄)alkyl group. The preferred dialkyl azodicarboxylate is diisopropyl azodicarboxylate (DIAD).

The Mitsunobu reaction is preferably carried out at room temperature.

In a particular embodiment, the compound of formula (III) may be represented by formula (IX) below: 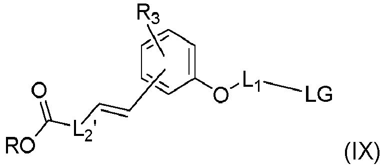
wherein L₁ and R₃ are as defined above in connection with the compound of formula (I),
LG is a leaving group, preferably a halogen such as a bromine atom,
R is a (C₁-C₄)alkyl group, preferably a methyl or ethyl group, more preferably a methyl group, and
L₂' is a bond, a (C₁-C₁₀) alkylene, a (C₂-C₁₀) alkenylene,

In this particular embodiment, the compound of formula (IX) may be prepared by a process comprising the steps of:
a) Subjecting a compound of formula (X) below: 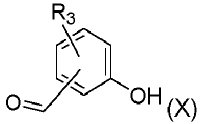
   with R₃ as defined above in connection with the compound of formula (I),
   to a Mitsunobu reaction with a compound of formula HO-L₁-LG (VIII) as defined above, to yield a compound formula (XI) below: 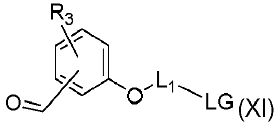
   with L₁ and R₃ is as defined above in connection with the compound of formula (I), and
b) Subjecting the compound of formula (XI) to a Wittig reaction with a compound of formula (XII):

   RO(O)C-L₂'-CH=P⁺Ar₄Ar₅Ar₆ X⁻ (XII)

   with Ar₄, Ar₅ and Ar₆ independently representing an aromatic group, preferably an aryl group, advantageously a phenyl group,
   R representing a (C1-C4)alkyl group, such as an ethyl or a methyl, preferably a methyl group, X⁻ representing a counter anion, such as an CH₃C(O)O⁻ (actetate) group, and
   L₂' as defined above.

The Mitsunobu reaction step a) may be performed as described above in connection with the compounds of formula (VII) and (VIII).

The Wittig reaction is preferably performed in an aprotic solvent such as toluene, typically at room temperature.

In a particular embodiment, the compound of formula (I) may be represented by formula (XIII) below: 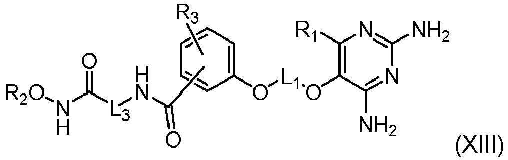 wherein L₁, R₁ R₂ and R₃ are as defined above in connection with the compound of formula (I), and
L₃ is a (C₁-C₁₀) alkylene or a (C₂-C₁₀) alkenylene.

In this particular embodiment, the compound of formula (XIII) may be prepared by a process comprising the steps of:
a) Subjecting a compound of formula (II) as defined above to a Williamson ether synthesis with a compound of formula (XIV) below: 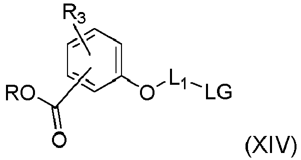
   with R₃ and L₁ as defined above in connection with the compounds of formula (I), and LG representing a leaving group,
   to obtain a compound of formula (XV) below 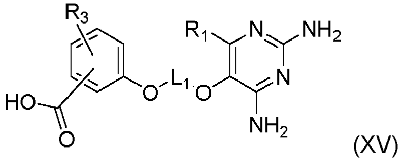
   with R₁, R₃, and L₁ as defined above in connection with the compounds of formula (I), and
b) Subjecting the compound of formula (XV) obtained in step a) to a peptidic coupling with a compound formula (XVI) below:

   H₂N-L₃-C(O)NH-OPG (XVI)

   wherein L₃ is a (C₁-C₁₀) alkylene or a (C₂-C₁₀) alkenylene, and
   PG is a THP protecting group or a (C₁-C₆)alkyl group, or a (C₁-C₆)alkylaryl group, said alkylaryl being optionally substituted by -OH, -OR^{a}, -OC(O)R^{b}, with R^{a} and R^{b} independently representing a (C₁-C₆)alkyl group,
   or an acid addition salt of the compound formula (XVI), such as a hydrochloride or a trifluoroacetic acid salt thereof.

When PG is a THP protecting group, the process further comprises a step c) of deprotecting the THP protecting group, typically under acidic conditions such as by contacting the compound obtained in step b) with aqueous HCl in a polar organic solvent, such as tetrahydrofuran (THF).

When the process is carried out to prepare a compound of formula (XIII), wherein R₂ is H and L₂ is a (C₂-C₁₂) alkenylene, PG in the compound of formula (XVI) is a THP protecting group. The process then further comprises a step c) of deprotecting the THP protecting group (typically under acidic conditions), thereby yielding the compound of formula (I).

When the process is carried out to prepare a compound of formula (XIII), wherein R₂ is H and L₂ is a bond or a (C₁-C₁₂) alkylene, PG in the compound of formula (XVI) is typically a benzyl group, and the process further comprises a step c) of deprotecting the PG group by hydrogenation, typically using Pd/C under a H₂ atmosphere, thereby yielding the compound of formula (I).

The Williamson ether synthesis step a) and the peptidic coupling step b) may be carried out as described above.

### Compositions

In another aspect, the present invention relates to a pharmaceutical or veterinary composition comprising a compound of formula (I) as described herein, and a pharmaceutically or veterinary acceptable carrier. Preferably, the composition is a pharmaceutical composition. Said pharmaceutically or veterinary acceptable carrier is selected, according to the dosage form and mode of administration desired, from the typical excipients known to persons skilled in the art.

The pharmaceutical or veterinary compositions according to the invention can be administered parenterally (such as intravenously or intradermally), topically, orally or rectally.

The term "parenteral" as used herein includes subcutaneous, intravenous or intramuscular techniques. Injectable preparations, for example, sterile injectable aqueous or oleaginous suspensions, may be formulated according to the known art using suitable dispersing or wetting agents and suspending agents. For therapeutic purposes, formulations for parenteral administration may be in the form of aqueous or non-aqueous isotonic sterile injection solutions or suspensions.

Preferably, the compositions of the invention are administered via oral route.

Solid dosage forms for oral administration may include capsules, tablets, pills, powders, and granules. In such solid dosage forms, the compound is ordinarily combined with one or more adjuvants appropriate to the indicated route of administration. Such capsules or tablets can contain a controlled-release formulation as can be provided in a dispersion of active compound in hydroxypropylmethyl cellulose. In the case of capsules, tablets, and pills, the dosage forms can also comprise buffering agents such as sodium citrate, or magnesium or calcium carbonate or bicarbonate. Tablets and pills can additionally be prepared with enteric coatings. Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents. In a particular embodiment, the pharmaceutical or veterinary composition of the invention further comprises another one or more therapeutic compounds.

Another aspect of the present invention encompasses a combination of a compound of formula (I) as described above, with one or more other therapeutic compounds.

The present invention also relates to a kit comprising:
- a composition comprising at least one compound of formula (I) as described herein;
- a second composition comprising at least another therapeutic compound, and
- preferably instructions for using said kit.

The other compound known to date as a combined therapy to treat malaria is suitable as the other therapeutic compound.

The other therapeutic compound is preferably an antiplasmodial/antimalarial agent and/or an antibiotic compound.

The antibiotic compound is preferably selected from the group consisting of sulfadoxine, azithromycin, clindamycin and doxycycline.

Advantageously, the therapeutic compound is an antiplasmodial/antimalarial compound, typically selected from the group consisting of pyrimethamine (PYR), chloroquine, mefloquine, quinine, proguanil, atovaquone, artemether, lumefantrine, artenimol, piperaquine, artesunate.

### Therapeutic Use

In another aspect, the invention relates to a compound of formula (I) as defined above and below, the composition or the kit as defined above, for use as a drug. The drug is in particular for preventing or treating parasitic diseases, such as malaria.

The invention further relates to the use of the compound or composition or a kit of the invention for manufacturing a medicament for preventing or treating parasitic diseases, such as malaria.

In another aspect, the invention relates to a method for preventing or treating a parasitic disease such as malaria, comprising administering to a patient in need thereof an effective dose of a compound of formula (I) or of the composition as defined above.

In particular, said parasitic diseases are malaria, toxoplasmosis, and cryptosporidiosis, preferably malaria and toxoplasmosis.

In a particular embodiment, the parasitic disease is malaria, notably a form resistant to ACTs or PYR, or a multidrug resistant form of malaria.

The "effective dose" of a compound of the invention varies as a function of numerous parameters such as, for example, the route of administration and the weight, the age, the sex, the advancement of the pathology to be treated and the sensitivity of the subject or patient to be treated.

As used herein, "patient" or "subject" includes any mammal, and is preferably a human being.

### EXAMPLES

The following examples are given for purely illustrative purposes, and should not be construed as limiting the invention in any way.

### Abbreviations

- TEA: triethylamine
- THP: tetrahydropyranyl
- TLC: Thil Layer Chromatography
- MPLC: Medium Pressure Liquid Chromatography
- RT: Room temperature
- Boc₂O: Di-tert-butyl dicarbonate
- FACS: Fluorescence-activated cell sorting
- DPBS: Dulbecco's Phosphate Buffered Saline
- HCl: hydrochloride

### 1 Synthesis

### 1.1 Materials and Methods

All materials and reagents were purchased from Sigma-Aldrich Co. Ltd. and abcr GmbH. All solvents were analytically pure and dried before use. Thin layer chromatography was carried out on aluminum sheets coated with silica gel 60 F254 (Merck, Darmstadt, Germany). For column chromatography under normal pressure silica gel 60 (0.036-0.200 mm) was used. Final compounds were confirmed to be of >95% purity based on HPLC. Purity was measured by UV absorbance at 254 nm. The analytical HPLC consists of an XTerra RP18 column (3.5 µm, 3.9 mm × 100 mm) from the manufacturer Waters (Milford, MA, USA) and two LC-10AD pumps, a SPD-M10A VP PDA detector, and a SIL-HT autosampler, all from the manufacturer Shimadzu (Kyoto, Japan). For preparative tasks an XTerra RP18 column (7 µm, 19 mm × 150 mm) from the manufacturer Waters (Milford, MA, USA) and two LC-20AD pumps were used. The mobile phase was in all cases a gradient of methanol/ water (starting at 95% water going to 5% water).

Mass spectrometry analyses were performed with a Finnigan MAT710C (Thermo Separation Products, San Jose, CA, USA) for the ESI-MS spectra and with a LTQ (linear ion trap) Orbitrap XL hybrid mass spectrometer (Thermo Fisher Scientific, Bremen, Germany) for the HRMS-ESI (high resolution mass spectrometry) spectra. For the HRMS analyses the signal for the isotopes with the highest prevalence was given and calculated (³⁵Cl, ⁷⁹Br).

¹H NMR and ¹³C NMR spectra were taken on a Varian Inova 400 using deuterated chloroform and deuterated DMSO as solvent. Chemical shifts are referenced to the residual solvent signals. The following abbreviations and formulas for solvents and reagents were used: dimethylformamide (DMF), dimethylsulfoxide (DMSO), tetrahydrofuran (THF), dichloromethane (DCM), N,N-diisopropylethylamine (DIPEA), triethylamine (TEA), hydrochloric acid (HCl) and trifluoroacetic acid (TFA).

### 1.2 General synthetic methods

### Method A: synthesis of 2,4-diamino-6-ethyl-5-hydroxypyrimidine hydrochloride

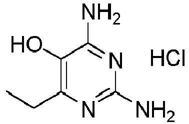

### Step 1: 2-amino-6-ethylpyrimidin-4-ol

In a microwave vial was placed potassium hydroxide (1.05 mmol), guanidine hydrochloride (1 mmol) and dry ethanol. The mixture was heated to 130 °C - with stirring- in a microwave reactor for 5 minutes to liberate the free base. After cooling, ethyl propionylacetate (1 mmol) was added, and the reaction was stirred again at 130 °C in a microwave reactor for 3 hours. The solvent was then evaporated under reduced pressure, and the residue was triturated from water. The obtained white precipitate was filtered, washed with water and dried.

MS m/z: 140.57 [M+H]⁺

Yield: 85%

¹H NMR (400 MHz, DMSO) δ 10.62 (s, 1H), 6.44 (s, 2H), 5.36 (s, 1H), 2.24 (q, *J* = 7.5 Hz, 2H), 1.06 (t, *J* = 7.5 Hz, 3H).

### Step 2: 2-amino-4-chloro-6-ethylpyrimidine

Phosphorous oxychloride (15 mmol) was added dropwise with stirring to solid 2-amino-6-ethylpyrimidin-4-ol (1 mmol) at 0 °C. After complete addition the suspension was refluxed for 30 minutes, and then phosphorous oxychloride was concentrated under reduced pressure. The residue obtained was added dropwise to ice-water mixture, with strong stirring. The mixture was then neutralized with ammonia solution while still at 0 °C. The obtained precipitate was stirred for 30 minutes, filtered, washed with cold water - ammonia mixture (1:1) and dried. MS m/z: 158.00 [M+H]⁺

Yield: 62%

¹H NMR (400 MHz, DMSO) δ 6.96 (s, 2H), 6.55 (s, 1H), 2.48 - 2.45 (m, 2H), 1.13 (t, *J* = *7.6* Hz, 3H).

### Step 3: 2,4-diamino-6-ethylpyrimidine

To solid 2-amino-4-chloro-6-ethylpyrimidine (1 mmol) in a microwave vial was added dropwise 7 N methanolic ammonia solution (2 ml) at 0 °C. The vial was then heated -with stirring- in a microwave reactor at 160 °C for 3 hours. Solvent was then evaporated under reduced pressure, and the obtained residue was triturated from a mixture of petroleum ether: ethyl acetate (1:1). The obtained precipitate was filtered, washed with diethyl ether and dried.

MS m/z: 139.10 [M+H]⁺

Yield: 97%

¹H NMR (400 MHz, DMSO) δ 7.15 (s, 2H), 6.71 (s, 2H), 5.72 (s, 1H), 2.38 (q, *J* = 7.6 Hz, 2H), 1.11 (t, *J* = 7.6 Hz, 3H).

### Step 4: 2,4-diamino-6-ethyl-5-hydroxypyrimidine hydrochloride

To a stirred suspension of 2,4-diamino-6-ethylpyrimidine (1 mmol) in 5 N sodium hydroxide (1.5 ml) at 0 °C was added slowly a solution of ammonium persulfate (1.5 mmol) in 0.5 ml water. The mixture was then stirred at room temperature for 16 h. The reaction was filtered, neutralized with concentrated HCl, and the precipitate formed was filtered, washed with cold water and dried to yield 2,4-diamino-6-ethyl-5-pyrimidyl hydrogen sulphate which was used directly for the next step.

2,4-Diamino-6-ethyl-5-pyrimidyl hydrogen sulphate (10 mmol) was stirred under reflux in 5 M HCl (10 ml) for 1 hour. The mixture was then allowed to stir in ice bath for another 1 hour. The separated solid was filtered, washed with diethyl ether and dried. The product needed no further purification.

MS m/z: 155.34 [M+H]⁺

Yield: 63% (over two steps)

¹H NMR (400 MHz, DMSO) δ 11.75 (s, 1H), 8.89 (s, 1H), 8.14 (s, 1H), 7.70 (s, 1H), 7.07 (s, 2H), 2.54 (q, *J* = 7.6 Hz, 2H), 1.12 (t, *J* = 7.6 Hz, 3H).

### Method B: esterification of carboxylic acids

The appropriate carboxylic acid (10 mmol) was dissolved in methanol and cooled in ice bath. Thionyl chloride (30 mmol) was added dropwise to the cooled solution while stirring. After complete addition the reaction mixture was left to attain room temperature, and then heated under reflux for 1 h. Solvent and excess thionyl chloride were evaporated under reduced pressure and the residue was extracted with ethyl acetate, washed with 1M sodium bicarbonate solution and brine. The organic solvent was dried over sodium sulfate and evaporated under reduced pressure to yield the product, which required no further purification.

### Method C: synthesis of 2-hydroxy-4-substitutedphenylpropanoic acids

To stirred formic acid (27 mmol) at 0 °C was added dropwise TEA (11 mmol) to give triethylammonium formate. This was added slowly to a mixture of the appropriate 2-hydroxy-4-substitutedbenzaldehyde (1 mmol) and Meldrum's acid (1 mmol), and the mixture was heated at 90-100 °C for 5 h. The reaction was allowed to cool, then basified with 1M sodium hydroxide solution. The aqueous layer was extracted with ethyl acetate and the organic layer was discarded. The aqueous layer was then acidified to pH 4 with 5M HCl solution and extracted with ethyl acetate. The organic layer was dried over anhydrous sodium sulfate, filtered, and concentrated in vacuo. The resultant slurry was purified by column chromatography (chloroform: methanol) to give the target compounds.

### Method D: Wittig reaction for synthesis of hydroxycinnamic acids

A mixture of the appropriate hydroxybenzaldehyde (1 mmol) and methyl (triphenylphosphoranylidene)acetate (1.1 mmol) in toluene was stirred at room temperature overnight. The solvent was then evaporated under reduced pressure, and the residue was purified by column chromatography (petroleum ether: ethyl acetate) to give the target compounds.

### Method E: Mitsunobu reaction

To a solution of the appropriate methyl ester (10 mmol) and triphenylphosphine (12 mmol) in dry THF at 0 °C was added dropwise diisopropyl azodicarboxylate (DIAD) (12 mmol). The solution was further stirred at 0 °C for 2 hours, then the appropriate bromo alcohol (12 mmol) was added dropwise, and the reaction was stirred overnight at room temperature. The solvent was then evaporated under reduced pressure, and the residue was triturated with n-hexane. The formed white precipitate of triphenylphosphine was filtered, washed with n-hexane and then discarded. The combined n-hexane extracts were evaporated under reduced pressure, and the product was purified using column chromatography (petroleum ether: ethyl acetate).

### Method F: synthesis of the carboxylic acids intermediates through Williamson ether synthesis followed by alkaline hydrolysis

A suspension of 2,4-diamino-6-ethyl-5-hydroxypyrimidine hydrochloride (5 mmol) and lithium hydroxide monohydrate (30 mmol) in DMF was stirred at room temperature for 30 min. To that suspension was added dropwise another well stirred suspension of the appropriate bromo ester (5 mmol) and potassium iodide (15 mmol) in DMF. The mixture was then stirred at room temperature until TLC indicated complete consumption of the starting materials. The solvent was then removed under reduced pressure, and the crude residue was dissolved in a minimum amount of water. This aqueous solution was then shaken with ethyl acetate, and the organic layer was discarded. The aqueous layer was neutralized with concentrated HCl.

Further workup depended on the purity of the separated product. When a solid separated, it was filtered, washed with water, and air dried to afford a pure product, which required no further purification. On the other hand, when the separated product was semi-solid, it was left in the refrigerator overnight, then the water was removed by decantation. The obtained residue was then dissolved in a mixture of ethyl acetate and THF, and the organic layer was shaken with brine, dried over sodium sulfate, and evaporated under reduced pressure. The product was used for the next step without further purification.

### Method G: synthesis of THP protected hydroxamic acids and deprotection

A mixture of the appropriate carboxylic acid (1 mmol), DIPEA (3 mmol) and NH₂OTHP (1.2 mmol) was stirred in dry THF, followed by the addition of PyBOP^{®} (1.2 mmol) and stirring was continued for 2 h at room temperature. The solvent was then removed under reduced pressure and the residue was dissolved in ethyl acetate, then shaken with water and brine. The organic layer was evaporated under vacuum and the product was purified by column chromatography (chloroform: methanol: TEA).

The obtained product was dissolved in THF followed by the addition of a catalytic amount of diluted HCl, and the mixture was stirred at room temperature overnight. The reaction was controlled by TLC. After that the solvent was evaporated under vacuum and the hydroxamic acid product was purified by reverse phase column chromatography (water: acetonitrile).

### Method H: synthesis of masked hydroxamic acids

The appropriate carboxylic acid (1 mmol.) was dissolved in dry THF, followed by the addition of HATU (1.2 mmol) and DIPEA (3 mmol). The mixture was stirred for 15 min, then the appropriate O-substitutedhydroxylamine (1.2 mmol) was added and the reaction mixture was stirred at room temperature for 2 h. The solvent was evaporated under vacuum and the mixture was dissolved in ethyl acetate and washed with aqueous sodium bicarbonate and ammonium chloride solutions then brine. The organic layer was separated, dried over anhydrous sodium sulfate, filtered and concentrated in vacuo. The residue was purified by column chromatography (chloroform: methanol).

O-Benzylhydroxylamine hydrochloride was commercially available, while 4-(aminooxymethyl)phenyl acetate was prepared as previously described (Ghazy, E. European Journal of Medicinal Chemistry, 2020. 200: p. 112338).

### Method I: amide coupling using HATU and catalytic dehydrogenation

A mixture of the appropriate carboxylic acid (1 mmol) and HATU (1.2 mmol) was dissolved in dry DMF (5 mL) and stirred at room temperature for 30 min. The corresponding amine (1.1 mmol) and DIPEA (5 mmol) were added and the reaction mixture was stirred for 18 h at RT. The reaction mixture was diluted with ethyl acetate (15 mL) and washed with 1 N NH₄Cl and sat. NaHCOs, respectively. The organic extracts were washed with brine, dried over anhydrous Na₂SO₄, filtered and concentrated under vacuum. The residue was purified using MPLC (chloroform: methanol) to provide the **required product**

The obtained product was dissolved in methanol (10 mL) and stirred at RT with Pd/C (10% w/w) under hydrogen atmosphere. The progress of reaction was monitored by TLC which indicated the disappearance of the starting material. The catalyst was filtered off through celite and washed with methanol, and the filtrate was collected and concentrated under vacuum to give the corresponding product.

### Method J: O-alkylation followed by Appel reaction

A mixture of the appropriate phenolic compound (1 mmol), potassium carbonate (1.5 mmol), 3-brom-1-propanol (1.5 mmol) and potassium iodide (0.5 mmol) was refluxed in acetonitrile (20 ml) overnight. The reaction was then concentrated and added to ice. The mixture was then extracted with ethyl acetate. The organic layer was washed with brine, dried over anhydrous Na₂SO₄, filtered and concentrated under vacuum. The residue was pure enough for the next step.The obtained product (1 mmol) and tetrabromomethane (1.3 mmol) were dissolved in DCM (20 ml) at 0 °C, and to the resulting solution was added triphenylphosphine (1.2 mmol) portionwise. The mixture was stirred at room temperature overnight, then the solvent was evaporated under reduced pressure, and the residue was purified using MPLC (petroleum ether: EtOAc).

### Method K: synthesis of the common intermediate 7-amino-N-(benzyloxy)heptanamide

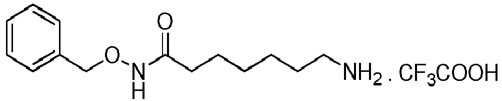

### Step 1; 7-f(tert-butoxycarbonyl)amino1heptanoic acid

A mixture of 7-aminoheptanoic acid (1 g, 6.89 mmol, 1.0 eq.) and NaOH (0.275 g, 6.89 mmol, 1.0 eq.) were dissolved in a dioxane-water (30 mL, 2:1), the mixture were cooled to 0 °C and di-tert-butyl dicarbonate Boc₂O (1.65 g, 7.57 mmol, 1.1 equiv ) was added in three equal portions. The reaction mixture was stirred at room temperature for 16 h and concentrated under reduced pressure. The resulting residue was dissolved in water (50 mL) and wash twice with ethyl acetate (50 mL). The aqueous layer was separated, acidified to pH 1-2 using aqueous 1 N HCl and extract with ethyl acetate (3 × 50 mL). The combined organic extracts were dried over anhydrous Na₂SO₄, filtered and concentrated under reduced pressure to afford the desired Boc-protected aminoheptanoic acid. ¹H NMR (400 MHz, DMSO): δ 11.92 (s, 1H), 6.72 (s, 1H), 2.86 (m, 2H), 2.16 (t, *J* = 7.4 Hz, 2H), 1.40 - 1.54 (m, 2H), 1.29 - 1.39 (m, 11H), 1.13 - 1.28 (m, 4H). Yield 88.8% (1.5 g, 6.11 mmol)

### Step 2; tert-butyl {7-f(benzyloxy)amino1-7-oxoheptyl}carbamate

To a solution of the Boc-protected-aminoheptanoic acid (1 g, 4.07 mmol, 1 eq.) in DMF (10 mL) was added HBTU (1.62 g, 4.28 mmol, 1.05 eq.), TEA (0.85 mL, 6.11 mmol, 1.5 eq.) and 4-dimethlyaminopyridine (0.124 g, 1.02 mmol, 0.25 eq.) followed by O-benzylbydroxylamine hydrochloride (0.65 g, 4.07 mmol, 1 eq). The reaction mixture was stirred for 16 h at room temperature, then quenched by adding water (30 mL). The aqueous layer was extracted with ethyl acetate (3 × 30 mL). The combined organic extract was washed with aqueous 1 N HCl (2 × 30 mL), sodium bicarbonate (2 × 30 mL), water (2 × 30 mL). The combined organic extracts were dried over anhydrous Na₂SO₄, filtered and concentrated under reduced pressure. The crude product was purified by Medium pressure liquid chromatography (MPLC). ¹H NMR (400 MHz, DMSO) δ 10.89 (s, 1H), 7.30 - 7.39 (m, 5H), 6.72 (s, 1H), 4.75 (s, 2H), 2.82 - 2.90 (m, 2H), 1.91 (m, 2H), 1.44 (m, 2H), 1.25 - 1.39 (m, 12H), 1.18 (s, 3H). Yield 52.5 % (0.75 g, 2.14 mmol).

### Step 3; 7-Amino-N-(benzyloxy)heptanamide

TFA (3 ml) was added to a solution of tert-butyl (7-((benzyloxy)amino)-7-oxoheptyl)carbamate (0.75, 2.14 mmol) in DCM (6 mL) at 0 °C. The reaction mixture was stirred at room temperature for 1 h, then it was concentrated under reduced pressure to afford the title compound as TFA salt. ¹H NMR (400 MHz, DMSO) δ 10.92 (s, 1H), 7.66 (s, 2H), 7. 27 - 7.42 (m, 5H), 4.76 (s, 2H), 2.80 - 2.68 (m, 2H), 1.93 (t, J = 7.3 Hz, 2H), 1.56 - 1.40 (m, 4H), 1.32 - 1.13 (m, 4H). Yield 91.74 % (1 g, 2.74 mmol).

### 1.3 Synthesis

### 3-(2-[3-(2,4-Diamino-6-ethylpyrimidin-5-yloxy)propoxy]phenyl)-N-hydroxypropanamide hydrochloride (compound 1 hydrochloride)

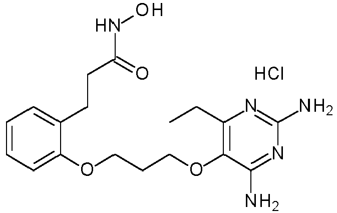

This compound was synthesized starting from 3-(2-hydroxyphenyl)propanoic acid which was converted to its methyl ester using method B. This ester was then reacted with 3-brom-1-propanol using method E to afford methyl 3-[2-(3-bromopropoxy)phenyl]propanoate, which was reacted then with 2,4-diamino-6-ethyl-5-hydroxypyrimidine hydrochloride using method F to yield the carboxylic acid intermediate 3-{2-[3-(2,4-diamino-6-ethylpyrimidin-5-yloxy)propoxy]phenyl}propanoic acid. This acid was finally converted to the hydroxamic acid using method G.

MS m/z: 376.18 [M+H]⁺

¹H NMR (400 MHz, DMSO) δ 12.47 (s, 1H,), 10.36 (s, 1H), 8.64 (s, 1H), 8.28 (s, 1H), 7.81 (s, 1H), 7.42 (s, 2H), 7.20 - 7.06 (m, 2H), 6.95 (d, *J* = 8.0 Hz, 1H), 6.84 (t, *J* = 7.4 Hz, 1H), 4.12 (t, *J* = 5.9 Hz, 2H), 3.90 (t, *J* = 6.2 Hz, 2H), 2.79 - 2.73 (m, 2H), 2.55 - 2.51 (m, 2H), 2.26 - 2.13 (m, 4H), 1.10 (t, *J* = 7.6 Hz, 3H).

### 3-(2-[2-(2,4-Diamino-6-ethylpyrimidin-5-yloxy)ethoxy]phenyl)-N-hydroxypropanamide hydrochloride (compound 2 hydrochloride)

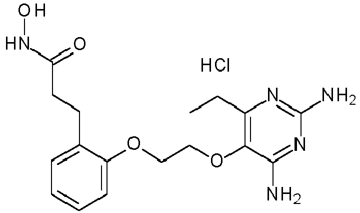

This compound was synthesized starting from 3-(2-hydroxyphenyl)propanoic acid which was converted to its methyl ester using method B. This ester was then reacted with 2-bromethanol using method E to afford methyl 3-[2-(2-bromoethoxy)phenyl]propanoate, which was reacted then with 2,4-diamino-6-ethyl-5-hydroxypyrimidine hydrochloride using method F to yield the carboxylic acid intermediate 3-{2-[2-(2,4-diamino-6-ethylpyrimidin-5-yloxy)ethoxy]phenyl}propanoic acid. This acid was finally converted to the hydroxamic acid using method G.

MS m/z: 362.39 [M+H]⁺

¹H NMR (400 MHz, DMSO) δ 12.47 (s, 1H), 10.36 (s, 1H), 8.66 (s, 1H), 8.28-7.70 (br s or m, 2H), 7.40 (s, 2H), 7.20 - 7.09 (m, 2H), 6.95 (d, *J* = 8.1 Hz, 1H), 6.86 (t, *J* = 7.3 Hz, 1H), 4.26 (d, *J* = 2.4 Hz, 2H), 4.07 (s, 2H), 2.81 - 2.75 (m, 2H), 2.60 (q, *J* = 7.6 Hz, 2H), 2.25 - 2.20 (m, 2H), 1.16 (t, *J* = 7.6 Hz, 3H).

### 3-(2-[4-(2,4-Diamino-6-ethylpyrimidin-5-yloxy)butoxy]phenyl)-N-hydroxypropanamide hydrochloride (compound 3 hydrochloride)

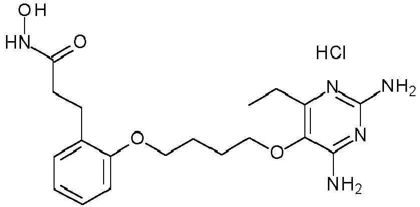

This compound was synthesized starting from 3-(2-hydroxyphenyl)propanoic acid which was converted to its methyl ester using method B. This ester was then reacted with 4-brom-1-butanol using method E to afford methyl 3-[2-(4-bromobutoxy)phenyl]propanoate, which was reacted then with 2,4-diamino-6-ethyl-5-hydroxypyrimidine hydrochloride using method F to yield the carboxylic acid intermediate 3-{2-[4-(2,4-diamino-6-ethylpyrimidin-5-yloxy)butoxy]phenyl}propanoic acid. This acid was finally converted to the hydroxamic acid using method G.

MS m/z: 390.59 [M+H]⁺

¹H NMR (400 MHz, DMSO) δ 12.40 (s, 1H), 10.35 (s, 1H), 8.63 (s, 1H), 8.27 (s, 1H), 7.77 (s, 1H), 7.41 (s, 2H), 7.17 - 7.06 (m, 2H), 6.92 (d, *J* = 7.9 Hz, 1H), 6.82 (t, *J* = 7.4 Hz, 1H), 4.00 (t, *J* = 5.8 Hz, 2H), 3.79 (t, *J* = 6.2 Hz, 2H), 2.80 - 2.72 (m, 2H), 2.55 (q, *J* = 7.6 Hz, 2H), 2.25 - 2.14 (m, 2H), 1.92 - 1.82 (m, 4H), 1.18 (t, *J* = 7.6 Hz, 3H).

### 3-(2-[3-(2,4-Diamino-6-ethylpyrimidin-5-yloxy)propoxy]-4-methoxyphenyl)-N-hydroxypropanamide hydrochloride (compound 4 hydrochloride)

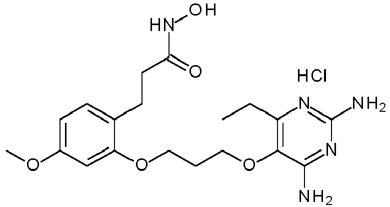

This compound was synthesized starting from 2-hydroxy-4-methoxybenzaldehyde which was converted to 3-(2-hydroxy-4-methoxyphenyl)propanoic acid using method C. This acid was then converted to its methyl ester using method B. This ester was then reacted with 3-brom-1-propanol using method E to afford methyl 3-[2-(3-bromopropoxy)-4-methoxyphenyl]propanoate, which was reacted then with 2,4-diamino-6-ethyl-5-hydroxypyrimidine hydrochloride using method F to yield the carboxylic acid intermediate 3-{2-[3-(2,4-diamino-6-ethylpyrimidin-5-yloxy)propoxy]-4-methoxyphenyl}propanoic acid. This acid was finally converted to the hydroxamic acid using method G.

MS m/z: 406.29 [M+H]⁺

¹H NMR (400 MHz, DMSO) δ 12.18 (s, 1H), 10.33 (s, 1H), 8.64 (s, 1H), 8.29 (s, 1H), 7.82 (s, 1H), 7.35 (s, 2H), 7.11 (d, *J =* 8.1 Hz, 1H), 7.03 (d, *J =* 1.9 Hz, 1H), 6.90 (dd, *J* = 8.0, 1.9 Hz, 1H), 4.15 (t, *J=* 5.9 Hz, 2H), 3.88 (t, *J* = 6.2 Hz, 2H), 3.70 (s, 3H), 2.78 - 2.68 (m, 2H), 2.54-2.49 (m, 2H), 2.19 (m, 4H), 1.10 (t, *J=* 7.6 Hz, 3H).

### 3-(4-Chloro-2-[3-(2,4-diamino-6-ethylpyrimidin-5-yloxy)propoxy]phenyi)-N-hydroxypropanamide hydrochloride (compound 5 hydrochloride)

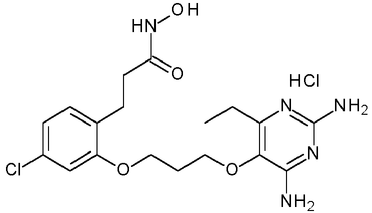

This compound was synthesized starting from 4-chloro-2-hydroxybenzaldehyde which was converted to 3-(4-chloro-2-hydroxyphenyl)propanoic acid using method C. This acid was then converted to its methyl ester using method B. This ester was then reacted with 3-brom-1-propanol using method E to afford methyl 3-[2-(3-bromopropoxy)-4-chlorophenyl]propanoate, which was reacted then with 2,4-diamino-6-ethyl-5-hydroxypyrimidine hydrochloride using method F to yield the carboxylic acid intermediate 3-{4-chloro-2-[3-(2,4-diamino-6-ethylpyrimidin-5-yloxy)propoxy]phenyl}propanoic acid. This acid was finally converted to the hydroxamic acid using method G.

MS m/z: 410.46 [M+H]⁺

¹H NMR (400 MHz, DMSO) δ 12.41 (s, 1H), 10.32 (s, 1H), 8.29 (s, 1H), 7.81 (s, 1H), 7.41 (s, 2H), 6.99 (d, *J* = 8.3 Hz, 1H), 6.52 (d, *J* = 2.3 Hz, 1H), 6.41 (dd, *J* = 8.3, 2.3 Hz, 1H), 4.10 (t, *J* = 5.9 Hz, 2H), 3.89 (t, *J* = 6.2 Hz, 2H), 2.71 - 2.64 (m, 2H), 2.55-2.49 (m, 2H), 2.22 - 2.11 (m, 4H), 1.11 (t, *J* = 7.6 Hz, 3H).

### N-(4-Acetoxybenzyloxy)-3-(2-(3-(2,4-diamino-6-ethylpyrimidin-5-yloxy)propoxy)phenyl)propenamide (compound 6)

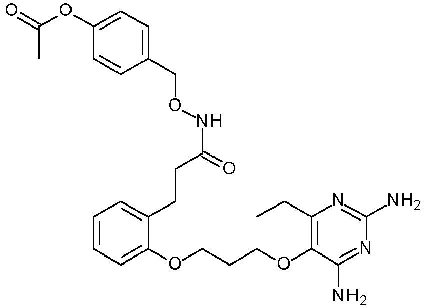

This compound was synthesized starting from 3-(2-hydroxyphenyl)propanoic acid which was converted to its methyl ester using method B. This ester was then reacted with 3-brom-1-propanol using method E to afford methyl 3-[2-(3-bromopropoxy)phenyl]propanoate, which was reacted then with 2,4-diamino-6-ethyl-5-hydroxypyrimidine hydrochloride using method F to yield the carboxylic acid intermediate 3-{2-[3-(2,4-diamino-6-ethylpyrimidin-5-yloxy)propoxy]phenyl}propanoic acid. This acid was finally converted to the final compound using method H.

MS m/z: 524.58 [M+H]⁺

¹H NMR (400 MHz, DMSO) δ 10.92 (s, 1H), 7.33 (d, *J* = 8.5 Hz, 2H), 7.19 - 7.06 (m, 4H), 6.97 (d, *J* = 8.1 Hz, 1H), 6.83 (t, *J* = 7.1 Hz, 1H), 6.50 (s, 2H), 5.90 (s, 2H), 4.69 (s, 2H), 4.14 (t, *J* = 6.0 Hz, 2H), 3.81 (t, *J* = 6.0 Hz, 2H), 2.78 (t, *J* = 7.5 Hz, 2H), 2.36 (q, *J* = 7.5 Hz, 2H), 2.29 - 2.10 (m, 7H), 1.01 (t, *J* = 7.5 Hz, 3H).

### N-(Benzyloxy)-3-(2-(3-(2,4-diamino-6-ethylpyrimidin-5-yloxy)propoxy)phenyl)propanamide (compound 7)

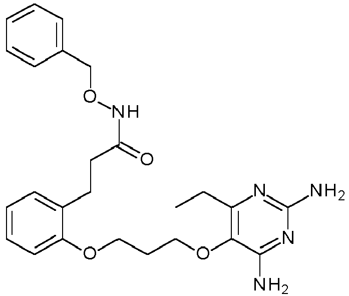

This compound was synthesized starting from 3-(2-hydroxyphenyl)propanoic acid which was converted to its methyl ester using method B. This ester was then reacted with 3-brom-1-propanol using method E to afford methyl 3-[2-(3-bromopropoxy)phenyl]propanoate, which was reacted then with 2,4-diamino-6-ethyl-5-hydroxypyrimidine hydrochloride using method F to yield the carboxylic acid intermediate 3-{2-[3-(2,4-diamino-6-ethylpyrimidin-5-yloxy)propoxy]phenyl}propanoic acid. This acid was finally converted to the final compound using method H.

MS m/z: 466.73 [M+H]⁺

¹H NMR (400 MHz, DMSO) δ 10.90 (s, 1H), 7.37 - 7.29 (m, 5H), 7.20 - 7.08 (m, 2H), 6.98 (d, *J=* 8.1 Hz, 1H), 6.84 (t, *J=* 7.0 Hz, 1H), 6.05 (s, 2H), 5.53 (s, 2H), 4.71 (s, 2H), 4.15 (t, *J=* 6.1 Hz, 2H), 3.79 (t, *J=* 6.0 Hz, 2H), 2.79 (t, *J =* 7.5 Hz, 2H), 2.33 (q, *J =* 7.5 Hz, 2H), 2.25 - 2.11 (m, 4H), 1.01 (t, *J* = 7.5 Hz, 3H).

### 3-[3-(2,4-Diamino-6-ethylpyrimidin-5-yloxy)propoxy]-N-hydroxybenzamide hydrochloride (compound 8 hydrochloride)

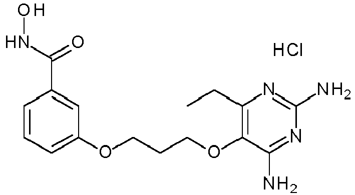

This compound was synthesized starting from methyl 3-hydroxybenzoate which was then reacted with 3-brom-1-propanol using method E to afford methyl 3-(3-bromopropoxy)benzoate, which was reacted then with 2,4-diamino-6-ethyl-5-hydroxypyrimidine hydrochloride using method F to yield the carboxylic acid intermediate 3-[3-(2,4-diamino-6-ethylpyrimidin-5-yloxy)propoxy]benzoic acid. This acid was finally converted to the hydroxamic acid using method G.

MS m/z: 348.33 [M+H]⁺

¹H NMR (400 MHz, DMSO) δ 12.40 (s, 1H), 11.19 (s, 1H), 9.00 (s, 1H), 8.30 (s, 1H), 7.82 (s, 1H), 7.55 - 7.22 (m, 5H), 7.16 - 6.97 (m, 1H), 4.17 (t, *J* = 6.0 Hz, 2H), 3.87 (t, *J* = 6.2 Hz, 2H), 2.52-2.50 (m, 2H), 2.23 - 2.15 (m, 2H), 1.09 (t, *J* = 7.6 Hz, 3H).

### 4-Chloro-3-[3-(2,4-diamino-6-ethylpyrimidin-5-yloxy)propoxy]-N-hydroxybenzamide (compound 9)

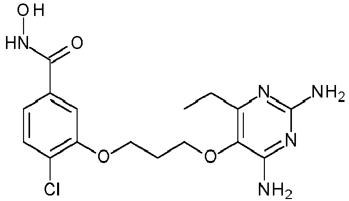

This compound was synthesized starting from 4-chloro-3-hydroxybenzoic acid which was converted to its methyl ester using method B. This ester was then reacted with 3-brom-1-propanol using method E to afford methyl 3-(3-bromopropoxy)-4-chlorobenzoate, which was reacted then with 2,4-diamino-6-ethyl-5-hydroxypyrimidine hydrochloride using method F to yield the carboxylic acid intermediate 2-chloro-5-[3-(2,4-diamino-6-ethylpyrimidin-5-yloxy)propoxy]benzoic acid. This acid was finally converted to the hydroxamic acid using method G.

MS m/z: 382.43 [M+H]⁺

¹H NMR (400 MHz, DMSO) δ 11.27 (s, 1H), 9.09 (s, 1H), 7.66 (s, 2H), 7.55 - 7.46 (m, 2H), 7.34 (dd, *J* = 8.2, 1.8 Hz, 1H), 6.86 (s, 2H), 4.28 (t, *J* = 5.9 Hz, 2H), 3.88 (t, *J* = 6.1 Hz, 2H), 2.46 - 2.39 (m, 2H), 2.28 - 2.17 (m, 2H), 1.03 (t, *J* = 7.6 Hz, 3H).

### 3-(3-[2,4-Diamino-6-ethylpyrimidin-5-yloxy)propoxy]-4-fluoro-N-hydroxybenzamide (compound 10)

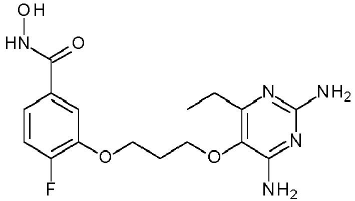

This compound was synthesized starting from 4- fluoro-3-hydroxybenzoic acid which was converted to its methyl ester using method B. This ester was then reacted with 3-brom-1-propanol using method E to afford methyl 3-(3-bromopropoxy)-4-fluorobenzoate, which was reacted then with 2,4-diamino-6-ethyl-5-hydroxypyrimidine hydrochloride using method F to yield the carboxylic acid intermediate 5-[3-(2,4-diamino-6-ethylpyrimidin-5-yloxy)propoxy]-2-fluorobenzoic acid. This acid was finally converted to the hydroxamic acid using method G.

MS m/z: 366.31 [M+H]⁺

¹H NMR (400 MHz, DMSO) δ 11.21 (s, 1H), 9.04 (s, 1H), 7.76 (s, 2H), 7.56 (dd, *J* = 8.3, 1.9 Hz, 1H), 7.40 - 7.33 (m, 1H), 7.32-7.25 (m, 1H), 7.08 (s, 2H), 4.26 (t, *J* = 6.0 Hz, 2H), 3.86 (t, *J* = 6.1 Hz, 2H), 2.48 - 2.40 (m, 2H), 2.26 - 2.16 (m, 2H), 1.06 (t, *J* = 7.6 Hz, 3H).

### N-(4-Acetoxybenzyloxy)-3-[3-(2,4-diamino-6-ethylpyrimidin-5-yloxy)propoxy]benzamide (compound 11)

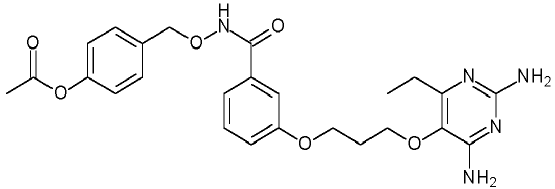

This compound was synthesized starting from methyl 3-hydroxybenzoate which was then reacted with 3-brom-1-propanol using method E to afford methyl 3-(3-bromopropoxy)benzoate, which was reacted then with 2,4-diamino-6-ethyl-5-hydroxypyrimidine hydrochloride using method F to yield the carboxylic acid intermediate 3-[3-(2,4-diamino-6-ethylpyrimidin-5-yloxy)propoxy]benzoic acid. This acid was finally converted to the final compound using method H.

MS m/z: 496.54 [M+H]⁺

¹H NMR (400 MHz, DMSO) δ 11.75 (s, 1H), 7.87 (br s, 2H), 7.47 (d, *J* = 8.4 Hz, 2H), 7.40 - 7.29 (m, 3H), 7.17 - 7.01 (m, 5H), 4.90 (s, 2H), 4.18 (t, *J* = 6.0 Hz, 2H), 3.86 (t, *J* = 6.1 Hz, 2H), 2.45 (m, 2H), 2.25 (s, 3H), 2.23 - 2.12 (m, 2H), 1.06 (t, *J* = 7.6 Hz, 3H).

### N-(Benzyloxy)-3-[3-(2,4-diamino-6-ethylpyrimidin-5-yloxy)propoxy]benzamide (compound 12)

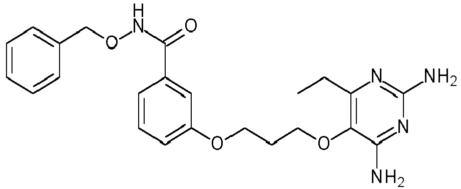

This compound was synthesized starting from methyl 3-hydroxybenzoate which was then reacted with 3-brom-1-propanol using method E to afford methyl 3-(3-bromopropoxy)benzoate, which was reacted then with 2,4-diamino-6-ethyl-5-hydroxypyrimidine hydrochloride using method F to yield the carboxylic acid intermediate 3-[3-(2,4-diamino-6-ethylpyrimidin-5-yloxy)propoxy]benzoic acid. This acid was finally converted to the final compound using method H.

MS m/z: 438.61 [M+H]⁺

¹H NMR (400 MHz, DMSO) δ 11.71 (s, 1H), 7.46 - 7.28 (m, 8H), 7.12 (dd, *J* = 7.4, 1.8 Hz, 1H), 6.29 (s, 2H), 5.73 (s, 2H), 4.91 (s, 2H), 4.18 (t, *J* = 6.1 Hz, 2H), 3.77 (t, *J* = 6.1 Hz, 2H), 2.34 (q, *J* = 7.5 Hz, 2H), 2.18 - 2.10 (m, 2H), 1.01 (t, *J* = 7.5 Hz, 3H).

### (E)-3-{2-[3-(2,4-Diamino-6-ethylpyrimidin-5-yloxy)propoxy]phenyl}-N-hydroxyacrylamide hydrochloride (compound 13 hydrochloride)

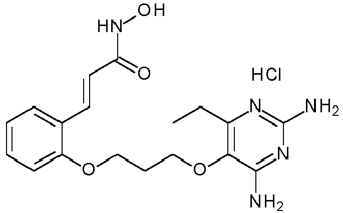

This compound was synthesized starting from 2-hydroxycinnamic acid which was converted to its methyl ester using method B. This ester was then reacted with 3-brom-1-propanol using method E to afford (E)-methyl 3-[2-(3-bromopropoxy)phenyl]acrylate, which was reacted then with 2,4-diamino-6-ethyl-5-hydroxypyrimidine hydrochloride using method F to yield the carboxylic acid intermediate (E)-3-{2-[3-(2,4-diamino-6-ethylpyrimidin-5-yloxy)propoxy]phenyl}acrylic acid. This acid was finally converted to the hydroxamic acid using method G.

MS m/z: 374.59 [M+H]⁺

¹H NMR (400 MHz, DMSO) δ 12.25 (s, 1H), 10.74 (s, 1H), 8.95 (s, 1H), 8.28 - 8.10 (br s, 1H), 7.85 (s, 1H), 7.66 (d, *J =* 15.9 Hz, 1H), 7.50 (d, *J=* 6.9 Hz, 1H), 7.34 (m, 3H), 7.10 (d, *J =* 8.3 Hz, 1H), 6.97 (t, *J* = 7.5 Hz, 1H), 6.52 (d, *J* = 15.9 Hz, 1H), 4.22 (t, *J* = 5.9 Hz, 2H), 3.89 (t, *J* = 5.9 Hz, 2H), 2.47 - 2.38 (m, 2H), 2.32 - 2.21 (m, 2H), 1.03 (t, *J* = 7.6 Hz, 3H).

### (E)-3-{2-[3-(2,4-Diamino-6-ethylpyrimidin-5-yloxy)propoxy]-4-methoxyphenyl}-N-hydroxyacrylamide hydrochloride (compound 14 hydrochloride)

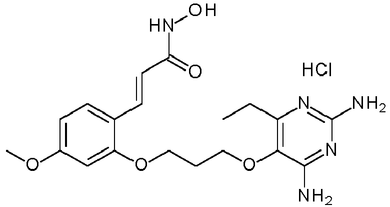

This compound was synthesized starting from 2-hydroxy-4-methoxybenzaldehyde which was converted to (E)-methyl 3-(2-hydroxy-4-methoxyphenyl)acrylate using method D. This ester was then reacted with 3-brom-1-propanol using method E to afford (E)-methyl 3-[2-(3-bromopropoxy)-4-methoxyphenyl]acrylate, which was reacted then with 2,4-diamino-6-ethyl-5-hydroxypyrimidine hydrochloride using method F to yield the carboxylic acid intermediate (E)-3-{2-[3-(2,4-diamino-6-ethylpyrimidin-5-yloxy)propoxy]-4-methoxyphenyl}acrylic acid. This acid was finally converted to the hydroxamic acid using method G.

MS m/z: 404.29 [M+H]⁺

¹H NMR (400 MHz, DMSO) δ 12.26 (s, 1H), 10.63 (s, 1H), 8.87 (s, 1H), 8.30 (s, 1H), 7.86 (s, 1H), 7.59 (d, *J* = 15.8 Hz, 1H), 7.48 - 7.31 (m, 3H), 6.64 (d, *J* = 2.2 Hz, 1H), 6.58 (dd, *J* = 8.6, 2.2 Hz, 1H), 6.40 (d, *J =* 15.9 Hz, 1H), 4.22 (t, *J =* 5.9 Hz, 2H), 3.89 (t, *J =* 6.1 Hz, 2H), 3.79 (s, 3H), 2.47 - 2.42 (m, 2H), 2.31 - 2.23 (m, 2H), 1.05 (t, *J* = 7.6 Hz, 3H).

### (E)-3-{4-Chloro-2-[3-(2,4-diamino-6-ethylpyrimidin-5-yloxy)propoxy]phenyl}-N-hydroxyacrylamide (compound 15)

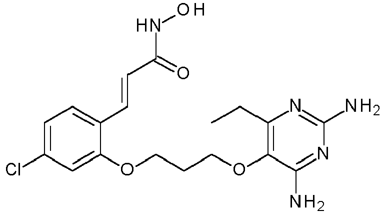

This compound was synthesized starting from 4-chloro-2-hydroxybenzaldehyde which was converted to (E)-methyl 3-(4-chloro-2-hydroxyphenyl)acrylate using method D. This ester was then reacted with 3-brom-1-propanol using method E to afford (E)-methyl 3-[2-(3-bromopropoxy)-4-chlorophenyl]acrylate, which was reacted then with 2,4-diamino-6-ethyl-5-hydroxypyrimidine hydrochloride using method F to yield the carboxylic acid intermediate (E)-3-{4-chloro-2-[3-(2,4-diamino-6-ethylpyrimidin-5-yloxy)propoxy]phenyl}acrylic acid. This acid was finally converted to the hydroxamic acid using method G.

MS m/z: 408.54 [M+H]⁺

¹H NMR (400 MHz, DMSO) δ 10.72 (s, 1H), 8.97 (s, 1H), 7.58 (d, *J =* 15.9 Hz, 1H), 7.51 (d, *J* = 8.3 Hz, 1H), 7.20 (d, *J* = 1.8 Hz, 1H), 7.03 (dd, *J* = 8.3, 1.9 Hz, 1H), 6.52 (d, *J =* 15.9 Hz, 1H), 6.09 (s, 2H), 5.54 (s, 2H), 4.26 (t, *J=* 6.0 Hz, 2H), 3.76 (t, *J* = 5.9 Hz, 2H), 2.33 - 2.16 (m, 4H), 0.93 (t, *J* = 7.5 Hz, 3H).

### (E)-N-(4-Acetoxybenzyloxy)-3-{2-[3-(2,4-diamino-6-ethylpyrimidin-5-yloxy)propoxy]phenyl}acrylamide (compound 16)

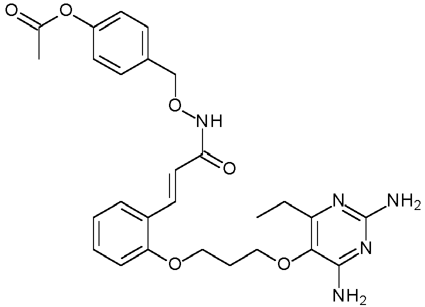

This compound was synthesized starting from 2-hydroxycinnamic acid which was converted to its methyl ester using method B. This ester was then reacted with 3-brom-1-propanol using method E to afford (E)-methyl 3-[2-(3-bromopropoxy)phenyl]acrylate, which was reacted then with 2,4-diamino-6-ethyl-5-hydroxypyrimidine hydrochloride using method F to yield the carboxylic acid intermediate (E)-3-{2-[3-(2,4-diamino-6-ethylpyrimidin-5-yloxy)propoxy]phenyl}acrylic acid. This acid was finally converted to the final compound using method H.

MS m/z: 522.52 [M+H]⁺

¹H NMR (400 MHz, DMSO) δ 11.24 (s, 1H), 7.76 - 7.65 (m, 1H), 7.51 (d, *J* = 7.4 Hz, 1H), 7.44 (d, *J* = 8.5 Hz, 2H), 7.38 - 7.30 (m, 1H), 7.15 - 7.08 (m, 3H), 6.99 - 6.94 (m, 1H), 6.65 - 6.39 (m, 3H), 5.93 (s, 2H), 4.85 (s, 2H), 4.23 (t, *J* = 6.0 Hz, 2H), 3.80 (t, *J =* 4.9 Hz, 2H), 2.37 - 2.16 (m, 7H), 0.96 (t, *J* = 7.6 Hz, 3H).

### (E)-N-(Benzyloxy)-3-{2-[3-(2,4-diamino-6-ethylpyrimidin-5-yloxy)propoxy]phenyl}acrylamide (compound 17)

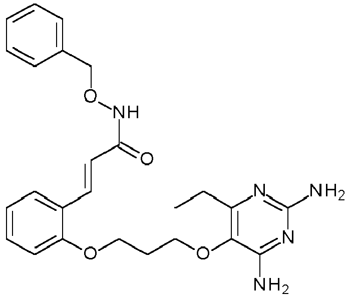

This compound was synthesized starting from 2-hydroxycinnamic acid which was converted to its methyl ester using method B. This ester was then reacted with 3-brom-1-propanol using method E to afford (E)-methyl 3-[2-(3-bromopropoxy)phenyl]acrylate, which was reacted then with 2,4-diamino-6-ethyl-5-hydroxypyrimidine hydrochloride using method F to yield the carboxylic acid intermediate (E)-3-{2-[3-(2,4-diamino-6-ethylpyrimidin-5-yloxy)propoxy]phenyl}acrylic acid. This acid was finally converted to the final compound using method H.

MS m/z: 464.44 [M+H]⁺

¹H NMR (400 MHz, DMSO) δ 11.22 (s, 1H), 7.73 (d, *J* = 15.9 Hz, 1H), 7.51 (d, *J* = 7.2 Hz, 1H), 7.42 - 7.29 (m, 6H), 7.11 (d, *J* = 8.2 Hz, 1H), 6.96 (t, *J* = 7.4 Hz, 1H), 6.48 (d, *J* = 15.9 Hz, 1H), 6.28 (s, 2H), 5.70 (s, 2H), 4.85 (s, 2H), 4.23 (t, *J* = 6.0 Hz, 2H), 3.79 (t, *J* = 5.4 Hz, 2H), 2.34 - 2.15 (m, 4H), 0.94 (t, *J* = 7.6 Hz, 3H).

### (E)-3-(3-[2-(2,4-Diamino-6-ethylpyrimidin-5-yloxy)ethoxy]phenyl)-N-hydroxyacrylamide hydrochloride (compound 18 hydrochloride)

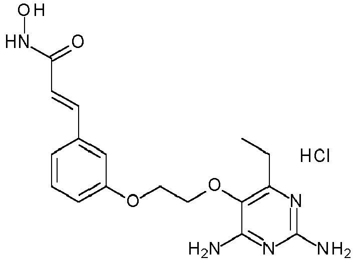

This compound was synthesized starting from 3-hydroxybenzaldehyde, which was converted to (E)-methyl 3-(3-hydroxyphenyl)acrylate using method D. This ester was then reacted with 2-bromethanol using method E to afford (E)-methyl 3-[3-(2-bromoethoxy)phenyl]acrylate, which was reacted then with 2,4-diamino-6-ethyl-5-hydroxypyrimidine hydrochloride using method F to yield the carboxylic acid intermediate (E)-3-{3-[2-(2,4-Diamino-6-ethylpyrimidin-5-yloxy)ethoxy]phenyl}acrylic acid. This acid was finally converted to the hydroxamic acid using method G.

MS m/z: 360.23 [M+H]⁺

¹H NMR (400 MHz, DMSO) δ 12.38 (s, 1H), 10.79 (s, 1H), 9.04 (s, 1H), 8.33 (s, 1H), 7.80 (s, 1H), 7.50 - 7.27 (m, 4H), 7.19 - 7.09 (m, 2H), 6.96 (dd, *J* = 8.1, 1.9 Hz, 1H), 6.48 (d, *J=* 15.8 Hz, 1H), 4.34-4.25 (m, 2H), 4.14 - 4.03 (m, 2H), 2.60 (q, *J=* 7.6 Hz, 2H), 1.15 (t, *J* = 7.6 Hz, 3H).

### (E)-3-{3-[3-(2,4-Diamino-6-ethylpyrimidin-5-yloxy)propoxy]phenyl}-N-hydroxyacrylamide hydrochloride (compound 19 hydrochloride)

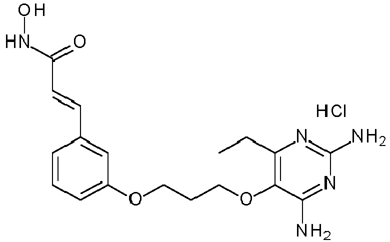

This compound was synthesized starting from 3-hydroxybenzaldehyde, which was converted to (E)-methyl 3-(3-hydroxyphenyl)acrylate using method D. This ester was then reacted with 3-brom-1-propanol using method E to afford (E)-methyl 3-[3-(3-bromopropoxy)phenyl]acrylate, which was reacted then with 2,4-diamino-6-ethyl-5-hydroxypyrimidine hydrochloride using method F to yield the carboxylic acid intermediate (E)-3-{3-[3-(2,4-diamino-6-ethylpyrimidin-5-yloxy)propoxy]phenyl}acrylic acid. This acid was finally converted to the hydroxamic acid using method G.

MS m/z: 374.31 [M+H]⁺

¹H NMR (400 MHz, DMSO) δ 12.54 (s, 1H), 10.75 (s, 1H), 8.30 (s, 1H), 7.81 (s, 1H), 7.50 - 7.26 (m, 5H), 7.16-7.08 (m, 2H), 6.95 (d, *J* = 7.6 Hz, 1H), 6.48 (d, *J* = 15.8 Hz, 1H), 4.16 (t, *J* = 5.2 Hz, 2H), 3.89 - 3.82 (m, 2H), 2.53-2.49 (m, 2H), 2.24 - 2.10 (m, 2H), 1.10 (t, *J=* 7.4 Hz, 3H).

### (E)-3-{4-[3-(2,4-Diamino-6-ethylpyrimidin-5-yloxy)propoxy]phenyl}-N-hydroxyacrylamide hydrochloride (compound 20 hydrochloride)

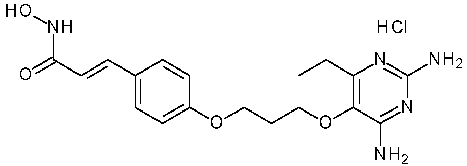

This compound was synthesized starting from 4-hydroxybenzaldehyde, which was converted to (E)-methyl 3-(4-hydroxyphenyl)acrylate using method D. This ester was then reacted with 3-brom-1-propanol using method E to afford (E)-methyl 3-[4-(3-bromopropoxy)phenyl]acrylate, which was reacted then with 2,4-diamino-6-ethyl-5-hydroxypyrimidine hydrochloride using method F to yield the carboxylic acid intermediate (E)-3-{4-[3-(2,4-diamino-6-ethylpyrimidin-5-yloxy)propoxy]phenyl}acrylic acid. This acid was finally converted to the hydroxamic acid using method G.

MS m/z: 374.29 [M+H]⁺

¹H NMR (400 MHz, DMSO) δ 12.59 (s, 1H), 10.71 (s, 1H), 8.31 (s, 1H), 7.82 (s, 1H), 7.53 - 7.34 (m, 6H), 6.97 (d, J = 8.8 Hz, 2H), 6.34 (d, J = 15.8 Hz, 1H), 4.16 (t, J = 6.0 Hz, 2H), 3.86 (t, J = 6.1 Hz, 2H), 2.53-2.49 (m, 2H), 2.22 - 2.13 (m, 2H), 1.10 (t, J = 7.6 Hz, 3H).

### (E)-3-(3-(3-[3-(2,4-Diamino-6-ethylpyrimidin-5-yloxy)propoxy]benzyloxy)phenyl)-N-hydroxyacrylamide hydrochloride (compound 21)

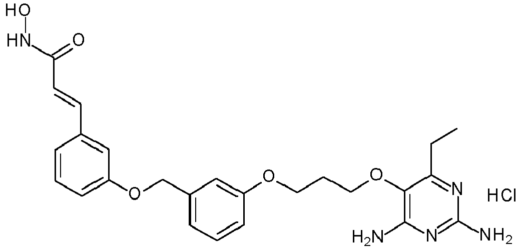

This compound was synthesized from (E)-methyl 3-(3-hydroxyphenyl)acrylate and [3-(3-bromopropoxy)phenyl]methanol using method E to afford (E)-methyl 3-{4-[3-(3-bromopropoxy)benzyloxy]phenyl}acrylate, which was reacted then with 2,4-diamino-6-ethyl-5-hydroxypyrimidine hydrochloride using method F to yield the carboxylic acid intermediate (E)-3-{3-[3-(2,4-diamino-6-ethylpyrimidin-5-yloxy)propoxy]phenyl}acrylic acid. This acid was finally converted to the hydroxamic acid using method G.
- (E)-Methyl 3-(3-hydroxyphenyl)acrylate was synthesized starting from 3-hydroxybenzaldehyde using method D.
- [3-(3-Bromopropoxy)phenyl]methanol was also synthesized starting with the reaction of 3-hydroxybenzaldehyde and 3-brom-1-propanol using method E to afford 3-(3-bromopropoxy)benzaldehyde, which was then reduced and brominated as previously described (Ghazy *et al.* European Journal of Medicinal Chemistry, 2020. **200:** p. 112338) to yield the required intermediate.

MS m/z: 480.40 [M+H]⁺

¹H NMR (400 MHz, DMSO) δ 12.31 (s, 1H), 10.77 (s, 1H), 8.92 (s, 1H), 8.30 (s, 1H), 7.82 (s, 1H), 7.43 - 7.25 (m, 5H), 7.19 - 7.10 (m, 2H), 7.04 - 6.88 (m, 4H), 6.47 (d, *J* = 15.8 Hz, 1H), 5.10 (s, 2H), 4.14 (t, *J* = 6.0 Hz, 2H), 3.86 (t, *J* = 6.2 Hz, 2H), 2.53 - 2.49 (m, 2H), 2.19 - 2.15 (m, 2H), 1.08 (t, *J* = 7.6 Hz, 3H).

### 4-(2-(3-[(2,4-Diamino-6-ethylpyrimidin-5-yl)oxy]propoxy)phenyl)-N-hydroxybutanamide (Compound 22)

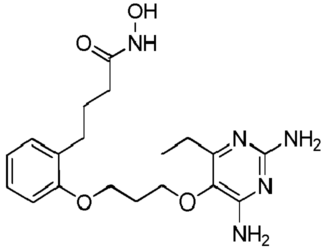

This compound was synthesized starting from the previously reported methyl 4-(2-hydroxyphenyl)butanoate (see WO 2015/179427) which was reacted with 3-brom-1-propanol using method E to afford methyl 4-[2-(3-bromopropoxy)phenyl]butanoate, which was then reacted with 2,4-diamino-6-ethyl-5-hydroxypyrimidine hydrochloride using method F to yield the carboxylic acid intermediate 4-(2-{3-[(2,4-diamino-6-ethylpyrimidin-5-yl)oxy]propoxy}phenyl)butanoic acid. This acid was finally converted to the corresponding hydroxamic acid through the reaction with *O*-benzylhydroxylamine hydrochloride using method I.

MS m/z: 389.70 [M+H]⁺

¹H NMR (400 MHz, DMSO) δ 10.31 (s, 1H), 8.80 (br. s, 1H), 7.19 - 7.05 (m, 2H), 6.95 (d, *J* = 8.1 Hz, 1H), 6.84 (t, *J* = 7.3 Hz, 1H), 6.32 (s, 2H), 5.76 (s, 2H), 4.12 (t, *J* = 6.0 Hz, 2H), 3.79 (t, *J* = 6.1 Hz, 2H), 2.57 - 2.52 (m, 2H), 2.34 (q, *J* = 7.5 Hz, 2H), 2.20 - 2.09 (m, 2H), 1.96 - 1.89 (m, 2H), 1.78 - 1.67 (m, 2H), 1.00 (t, *J* = 7.5 Hz, 3H).

### 5-(2-(3-[(2,4-Diamino-6-ethylpyrimidin-5-yl)oxy]propoxy)phenyl)-N-hydroxypentanamide (compound 23)

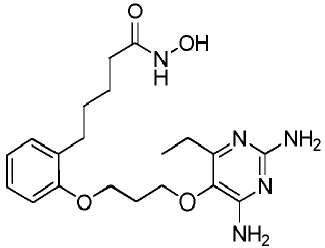

This compound was synthesized starting from the previously reported methyl 5-(2-hydroxyphenyl)pentanoate which was reacted with 3-brom-1-propanol using method E to afford 1-(3-bromopropoxy)-2-(5-methoxypentyl)benzene, which was then reacted with 2,4-diamino-6-ethyl-5-hydroxypyrimidine hydrochloride using method F to yield the carboxylic acid intermediate 5-(2-{3-[(2,4-diamino-6-ethylpyrimidin-5-yl)oxy]propoxy}phenyl)pentanoic acid.

This acid was finally converted to the corresponding hydroxamic acid through the reaction with O-benzylhydroxylamine hydrochloride using method I.

MS m/z: 403.70 [M+H]⁺

¹H NMR (400 MHz, DMSO) δ 10.30 (s, 1H), 8.71 (br. s, 1H), 7.19 - 7.04 (m, 2H), 6.94 (d, *J* = 8.0 Hz, 1H), 6.83 (t, *J* = 7.3 Hz, 1H), 6.14 (s, 2H), 5.61 (s, 2H), 4.12 (t, *J* = 6.0 Hz, 2H), 3.78 (t, *J* = 6.2 Hz, 2H), 2.55 - 2.51 (m, 2H), 2.38 - 2.24 (m, 2H), 2.21 - 2.07 (m, 2H), 1.96 - 1.90 (m, 2H), 1.50 - 1.43 (m, 4H), 1.00 (t, *J* = 7.5 Hz, 3H).

### 3-(2-{3-[(2,4-Diamino-6-cyclopropylpyrimidin-5-yl)oxy]propoxy}phenyl)-N-hydroxypropanamide (compound 24)

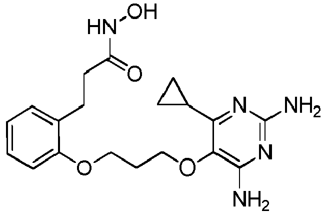

This compound was synthesized starting from 3-(2-hydroxyphenyl)propanoic acid which was converted to its methyl ester using method B. This ester was then reacted with 3-brom-1-propanol using method E to afford methyl 3-[2-(3-bromopropoxy)phenyl]propanoate, which was then reacted with 6-cyclopropyl-2,4-diamino-5-hydroxypyrimidine hydrochloride using method F to yield the carboxylic acid intermediate 3-(2-{3-[(2,4-diamino-6-cyclopropylpyrimidin-5-yl)oxy]propoxy}phenyl)propanoic acid. This acid was finally converted to the corresponding hydroxamic acid through the reaction with *O*-benzylhydroxylamine hydrochloride using method I.

The intermediate 6-cyclopropyl-2,4-diamino-5-hydroxypyrimidine hydrochloride was synthesized (as shown in scheme 1) starting from ethyl 3-cyclopropyl-3-oxopropanoate and using method A as previously described for 2,4-diamino-6-ethyl-5-hydroxypyrimidine hydrochloride

MS m/z: 387.80 [M+H]⁺

¹H NMR (400 MHz, DMSO) δ 10.32 (s, 1H), 8.64 (s, 1H), 7.19-7.02 (m, 2H), 6.95 (d, *J* = 8.1 Hz, 1H), 6.82 (t, *J* = 7.4 Hz, 1H), 6.00 (s, 2H), 5.41 (s, 2H), 4.15 (t, *J* = 6.0 Hz, 2H), 3.87 (t, *J* = 6.1 Hz, 2H), 2.80 - 2.71 (m, 2H), 2.25 - 2.12 (m, 4H), 2.01 - 1.91 (m, 1H),, 0.80 - 0.70 (m, 2H), 0.65 - 0.65 (m, 2H).

### 3-(3-(3-[(2,4-Diamino-6-ethylpyrimidin-5-yl)oxy]propoxy)phenyl)-N-hydroxypropanamide (compound 25)

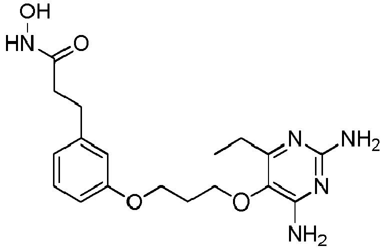

This compound was synthesized starting from 3-(3-hydroxyphenyl)propanoic acid which was converted to its methyl ester using method B. This ester was then reacted with 3-brom-1-propanol using method E to afford methyl 3-[3-(3-bromopropoxy)phenyl]propanoate, which was then reacted with 2,4-diamino-6-ethyl-5-hydroxypyrimidine hydrochloride using method F to yield the carboxylic acid intermediate 3-(3-{3-[(2,4-diamino-6-ethylpyrimidin-5-yl)oxy]propoxy}phenyl)propanoic acid. This acid was finally converted to the corresponding hydroxamic acid through the reaction with O-benzylhydroxylamine hydrochloride using method I.

MS m/z: 375.70 [M+H]⁺

¹H NMR (400 MHz, DMSO) δ 10.33 (s, 1H), 8.69 (s, 1H), 7.16 (t, *J* = 7.9 Hz, 1H), 6.81 - 6.71 (m, 3H), 6.06 (s, 2H), 5.54 (s, 2H), 4.11 (t, *J* = 6.1 Hz, 2H), 3.74 (t, *J* = 6.1 Hz, 2H), 2.76 (t, *J* = 7.7 Hz, 2H), 2.33 (q, *J* = 7.5 Hz, 2H), 2.23 (t, *J* = 7.7 Hz, 2H), 2.17 - 2.03 (m, 2H), 1.01 (t, *J* = 7.5 Hz, 3H).

### 3-(4-(3-[(2,4-Diamino-6-ethylpyrimidin-5-yl)oxy]propoxy)phenyl)-N-hydroxypropanamide (compound 26)

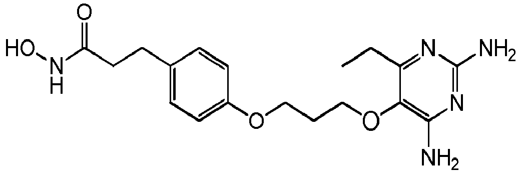

This compound was synthesized starting from 3-(4-hydroxyphenyl)propanoic acid which was converted to its methyl ester using method B. This ester was then reacted with 3-brom-1-propanol using method E to afford methyl 3-[4-(3-bromopropoxy)phenyl]propanoate, which was then reacted with 2,4-diamino-6-ethyl-5-hydroxypyrimidine hydrochloride using method F to yield the carboxylic acid intermediate 3-(4-{3-[(2,4-diamino-6-ethylpyrimidin-5-yl)oxy]propoxy}phenyl)propanoic acid. This acid was finally converted to the corresponding hydroxamic acid through the reaction with O-benzylhydroxylamine hydrochloride using method I.

MS m/z: 376.1 [M+H]⁺

¹H NMR (400 MHz, DMSO) δ 10.32 (s, 1H), 8.68 (s, 1H), 7.08 (d, *J* = 8.5 Hz, 2H), 6.84 (d, *J* = 8.6 Hz, 2H), 6.07 (s, 2H), 5.54 (s, 2H), 4.10 (t, *J* = 6.2 Hz, 2H), 3.74 (t, *J=* 6.1 Hz, 2H), 2.72 (t, *J* = 7.7 Hz, 2H), 2.32 (q, *J* = 7.5 Hz, 2H), 2.19 (t, *J* = 7.7 Hz, 2H), 2.14 - 2.05 (m, 2H), 1.01 (t, *J* = 7.5 Hz, 3H).

### 4-(3-[(2,4-Diamino-6-ethylpyrimidin-5-yl)oxy]propoxy)-N-hydroxybenzamide (compound 27)

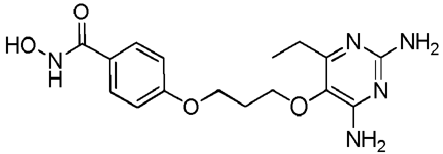

This compound was synthesized starting from methyl 4-hydroxybenzoate which was then reacted with 3-brom-1-propanol using method E to afford methyl 4-(3-bromopropoxy)benzoate, which was then reacted with 2,4-diamino-6-ethyl-5-hydroxypyrimidine hydrochloride using method F to yield the carboxylic acid intermediate 4-[3-(2,4-diamino-6-ethylpyrimidin-5-yloxy)propoxy]benzoic acid. This acid was finally converted to the corresponding hydroxamic acid through the reaction with O-benzylhydroxylamine hydrochloride using method I.

MS m/z: 347.9 [M+H]⁺

¹H NMR (400 MHz, DMSO) δ 11.03 (s, 1H), 8.88 (s, 1H), 7.71 (d, *J* = 8.8 Hz, 2H), 6.99 (d, *J* = 8.8 Hz, 2H), 6.28 (s, 2H), 5.72 (s, 2H), 4.19 (t, *J* = 6.1 Hz, 2H), 3.76 (t, *J* = 6.1 Hz, 2H), 2.33 (q, *J* = 7.5 Hz, 2H), 2.20 - 2.08 (m, 2H), 1.00 (t, *J* = 7.6 Hz, 3H).

### N¹-(2-{3-[(2,4-diamino-6-ethylpyrimidin-5-yl)oxy]propoxy}phenyl)-N⁶-hydroxyadipamide (compound 28)

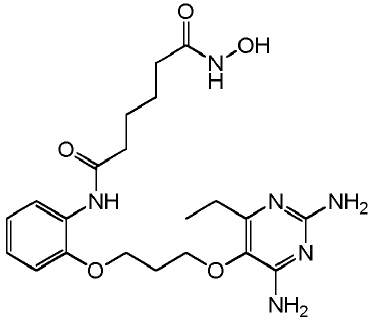

This compound was synthesized through an amide coupling reaction between 2-(benzyloxy)aniline and mono-methyl adipate followed by removal of the benzyl group using method I to afford methyl 6-[(2-hydroxyphenyl)amino]-6-oxohexanoate which was then converted to methyl 6-{[2-(3-bromopropoxy)phenyl]amino}-6-oxohexanoate using method J. This compound was then reacted with 2,4-diamino-6-ethyl-5-hydroxypyrimidine hydrochloride using method F to yield the carboxylic acid intermediate 6-[(2-{3-[(2,4-diamino-6-ethylpyrimidin-5-yl)oxy]propoxy}phenyl)amino]-6-oxohexanoic acid. This acid was finally converted to the corresponding hydroxamic acid through the reaction with *O-*benzylhydroxylamine hydrochloride using method I.

MS m/z: 447.0 [M+H]⁺

¹H NMR (400 MHz, DMSO) δ 10.33 (s, 1H), 8.79 (s, 1H), 8.70 (br. s, 1H), 7.89 (d, *J* = 7.8 Hz, 1H), 7.10 - 6.99 (m, 2H), 6.93 - 6.82 (m, 1H), 6.08 (s, 2H), 5.55 (s, 2H), 4.18 (t, *J* = 6.1 Hz, 2H), 3.80 (t, *J* = 6.1 Hz, 2H), 2.35 - 2.26 (m, 4H), 2.22 - 2.11 (m, 2H), 1.98 - 1.89 (m, 2H), 1.54 - 1.46 (m, 4H), 0.98 (t, *J* = 7.5 Hz, 3H).

### N¹-(2-(3-((2,4-diamino-6-ethylpyrimidin-5-yl)oxy)propoxy)phenyl)-N⁸-hydroxyoctanediamide (Compound 29)

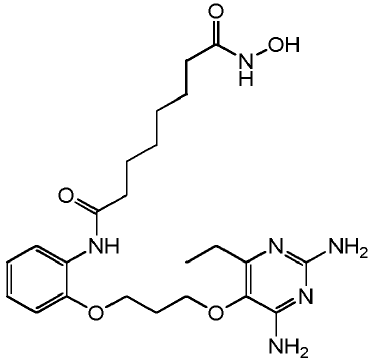

This compound was synthesized through an amide coupling reaction between 2-(benzyloxy)aniline and mono-methyl suberate followed by removal of the benzyl group using method I to afford methyl 8-[(2-hydroxyphenyl)amino]-8-oxoctanoate which was then converted to methyl 8-{[2-(3-bromopropoxy)phenyl]amino}-8-oxoctanoate using method J. This compound was then reacted with 2,4-diamino-6-ethyl-5-hydroxypyrimidine hydrochloride using method F to yield the carboxylic acid intermediate 8-[(2-{3-[(2,4-diamino-6-ethylpyrimidin-5-yl)oxy]propoxy}phenyl)amino]-8-oxoctanoic acid. This acid was finally converted to the corresponding hydroxamic acid through the reaction with *O-*benzylhydroxylamine hydrochloride using method I.

MS m/z: 475.4 [M+H]⁺

¹H NMR (400 MHz, DMSO) δ 10.30 (s, 1H), 8.78 (s, 1H), 8.70 (br. s, 1H), 7.89 (d, *J* = 7.8 Hz, 1H), 7.09 - 6.97 (m, 2H), 6.90 - 6.82 (m, 1H), 6.10 (s, 2H), 5.59 (s, 2H), 4.18 (t, *J* = 6.0 Hz, 2H), 3.80 (t, *J* = 6.1 Hz, 2H), 2.37 - 2.11 (m, 6H), 1.91 (t, *J* = 7.3 Hz, 2H), 1.56 - 1.42 (m, 4H), 1.29 - 1.12 (m, 4H), 0.98 (t, *J* = 7.6 Hz, 3H).

### 3-{3-[(2,4-Diamino-6-ethylpyrimidin-5-yl)oxy]propoxy}-N-[7-(hydroxyamino)-7-oxoheptyl]benzamide (Compound 30)

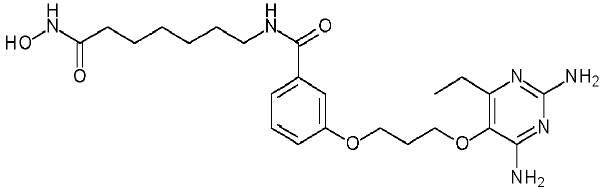

This compound was synthesized starting from methyl 3-hydroxybenzoate which was converted to methyl 3-(3-bromopropoxy)benzoate using method E which was reacted then with 2,4-diamino-6-ethyl-5-hydroxypyrimidine hydrochloride using method F to yield the carboxylic acid intermediate 3-[3-(2,4-diamino-6-ethylpyrimidin-5-yloxy)propoxy]benzoic acid. This acid was finally converted to the final hydroxamic acid through reaction with 7-amino-*N-*(benzyloxy)heptanamide followed by deprotection using method I.

MS m/z: 475.73 [M+H]⁺

¹H NMR (400 MHz, DMSO) δ 10.32 (s, 1H), 8.72 (s, 1H), 8.40 (t, *J* = 5.5 Hz, 1H), 7.26 - 7.46 (m, 3H), 7.02 - 7.14 (m, 1H), 6.85 (s, 2H), 6.25 (s, 2H), 4.19 (t, *J* = 6.1 Hz, 2H), 3.80 (t, *J* = 6.1 Hz, 2H), 3.18 - 3.23 (m, 2H), 2.38 (q, *J* = 7.6 Hz, 2H), 2.11 - 2.19 (m, 2H), 1.92 (t, *J* = 7.3 Hz, 2H), 1.43-1.53 (m, 4H), 1.22-1.31 (m, 4H), 1.03 (t, *J* = 7.6 Hz, 3H)

### 4-{3-[(2,4-Diamino-6-ethylpyrimidin-5-yl)oxy]propoxy}-N-[7-(hydroxyamino)-7-oxoheptyl]benzamide (Compound 31)

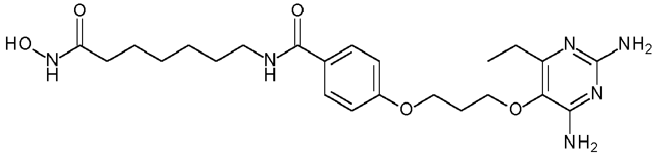

This compound was synthesized starting from methyl 4-hydroxybenzoate which was converted to methyl 4-(3-bromopropoxy)benzoate using method E which was reacted then with 2,4-diamino-6-ethyl-5-hydroxypyrimidine hydrochloride using method F to yield the carboxylic acid intermediate 4-[3-(2,4-diamino-6-ethylpyrimidin-5-yloxy)propoxy]benzoic acid. This acid was finally converted to the final hydroxamic acid through reaction with 7-amino-*N-*(benzyloxy)heptanamide followed by deprotection using method I.

MS m/z: 475.41 [M+H]⁺

¹H NMR (500 MHz, DMSO) δ 10.31 (s, 1H), 8.64 (s, 1H), 8.26 (t, *J* = 5.3 Hz, 1H), 7.80 (d, *J* = 8.7 Hz, 2H), 6.99 (d, *J* = 8.7 Hz, 2H), 6.72 (s, 2H), 6.10 (s, 2H), 4.20 (t, *J* = 6.0 Hz, 2H), 3.79 (t, *J* = 6.0 Hz, 2H), 3.18 - 3.23 (m, 2H), 2.37 (q, *J* = 7.6 Hz, 2H), 2.12 - 2.19 (m, 2H), 1.92 (t, *J* = 7.3 Hz, 2H), 1.43 - 1.53 (m, 4H), 1.22-1.31 (m, 4H), 1.02 (t, *J* = 7.5 Hz, 3H).

### 2 In vitro Plasmodium growth inhibition by dual-targeting compounds

### 2.1 Materials and Methods

### 2.1.1 Plasmodium falciparum growth inhibition assays

The capacity of 23 compounds to inhibit P. *falciparum* growth *in vitro* has been evaluated using a drug-sensitive strain (Pf3D7) and a multi resistant strain (PfDd2). Growth inhibition assays were performed using SYBR Green I as previously reported in Eur J Med Chem, 2019. 161: p. 277-291. Briefly, in vitro cultured asynchronous *P*. *falciparum* infected erythrocytes (0.5% parasitemia, 1% haematocrit) were seeded, in duplicate wells, into 96-well tissue culture plates containing vehicle control (DMSO), positive control (**CQ**) or test compound. Plates were incubated under standard *P. falciparum* culture conditions for 48 h. Cultures were stained for 30 min in the dark with SYBR Green I 1X (Invitrogen) diluted in 20 mM Tris pH 8.8, 138 mM NaCl, and fixed with 1% paraformaldehyde. Fixed parasitized red blood cells (RBC) were stored at 4 °C in the dark until flow cytometry analysis. Parasite growth was assessed by flow cytometry on a BD FACS Cantoll (BD Biosciences) or a Attune^{™} NxT Flow Cytometer (Invitrogen). Cell doublets were excluded from the analysis using a forward scatter (FSC)-width versus FSC-area dot plot. Infected and uninfected erythrocytes were gated on the basis of their FSC and side scatter (SSC) signals. Fluorescence analysis (Green fluorescence FITC) was performed using BD FACSDiva software (BD Biosciences) on a total of 200,000 acquired events. Fluorescence was observed as described by Izumiyama et al. (Exp Parasitol, 2009. 121(2): p. 144-50) on a two-parameter dot plot (FITC-FSC). Fluorescence of non-infected RBC was adjusted to plot between 10° and 10². Results are expressed as the percentage of growth inhibition.

Compounds were tested in 12-points dose response assays with 2-fold serial dilutions to determine 50% growth inhibition (IC₅₀) values. All IC₅₀ values were calculated using log-linear interpolation (www.antimalarial-icestimator.net), with mean values (± SD) determined over at least two independent experiments.

### 2.1.2 Cytotoxicity of dual-targeting compounds on HEK293 cells

Human embryonic kidney 293 (HEK293) cells (DSMZ Braunschweig, ACC305) were incubated at 37 °C in a humidified incubator with 5% CO2 in Dulbecco's modified Eagle medium (DMEM) supplemented with 10% FCS and 5 mM glutamine. Cells were seeded out at 1.5 × 103 cells per well in a 96-well cell culture plate (TPP, Switzerland). The compounds were added immediately to the medium at 50 µM or increasing concentrations to determine IC₅₀ values.

After 24 h, Alamar Blue reagent (Invitrogen, CA) was added according to the manufacturer's instructions and incubated again for 21 h before samples were analyzed. Detection of viable cells which convert the resazurine compound of reagent into the high fluorescent resorufin was performed by using a FLUOstar OPTIMA microplate reader (BMG Labtec) with the following filter set: Ex 560 nm/Em 590 nm.

All measurements were performed in triplicate, and data are the mean with SD ≤ 12%. As a positive control daunorubicin was used, and an IC₅₀ value of 12.55 ± 0.07 µM was determined.

2.2 Results : *in vitro* activity of dual-targeting compounds against drug sensitive Pf3D7 line Results of growth inhibition assays of Pf3D7 are presented in Table I. All dual-targeting compounds showed IC₅₀ lower than 20 nM. 18 compounds showed an IC₅₀ < 1nM. Among them, 6 compounds (**1, 4, 11, 9, 19, 20**) are extremely potent and showed an IC₅₀ <200 pM and 4 compounds (**13, 14, 15, 16**) showed an IC₅₀ < 100 pM. These IC₅₀ values are lower than the IC₅₀ of **CQ** which was 4.7 ± 1.7 nM under the same conditions.

**Table I. In vitro antiplasmodial activity of dual-targeting compounds against P. falciparum 3D7-infected erythrocytes. IC50 values are presented as mean ± SD with n ≥ 2.**

| **Compound n°** | MW (g/mol) | Pf3D7 IC₅₀ (nM) |
|---|---|---|
| **1·HCl** | 411.17 | 0.170 ± 0.018 |
| **2·HCl** | 397.85 | 11.4 ± 7.9 |
| **3·HCl** | 425.9 | 0.451 ± 0.088 |
| **4·HCl** | 441.9 | 0.173 ± 0.122 |
| **5·HCl** | 445.12 | 0.279 ± 0.154 |
| 6 | 523.24 | 0.169 ± 0.041 |
| **7** | 465.23 | 0.497 ± 0.055 |
| **8·HCl** | 383.83 | 0.845 ± 0.108 |
| **9** | 381.12 | 0.172 ± 0.027 |
| **10** | 401.13 | 0.390 ± 0.012 |
| **11** | 495.21 | 1.02 ± 0.68 |
| **12** | 437.2 | 4.836 ± 1.053 |
| **13·HCl** | 409.87 | 0.081 ± 0.043 |
| **14·HCl** | 439.16 | 0.088 ± 0.02 |
| **15** | 407.13 | 0.082 ± 0.007 |
| **16** | 521.56 | 0.067 ± 0.005 |
| **17** | 463.52 | 0.331 ± 0.124 |
| **18·HCl** | 395.13 | 0.249 ± 0.015 |
| **19·HCl** | 409.15 | 0.173 ± 0.021 |
| **21** | 515.98 | 0.347 ± 0.047 |
| **20·HCl** | 395.13 | 0.117 ± 0.045 |
| **30** | 474.55 | 0.876 ± 0.018 |
| **31** | 474.55 | 0.404 ± 0.136 |

### 2.3 Results : in vitro selectivity of dual targeting compounds for Pf3D7 vs HEK293

12 compounds were tested for their cytotoxicity against HEK293 cells. No toxicity was observed at 50 µM. This means that Selectivity Indexes (SI) on Pf3D7, calculated as the ratio of the minimum inhibitory concentration of HEK293 cells (50 µM) to the IC₅₀ of Pf3D7, are greater than 1.10⁶ (Table II).

**Table II. Compound selectivity for Pf3D7 versus mammalian HEK293 cells for selected compounds.**

| **Compound n°** | Pf3D7 IC₅₀(nM) | Selectivity Index HEK293 / Pf3D7 |
|---|---|---|
| **1·HCl** | 0.170 ± 0.018 | > 2.9E+08 |
| **2·HCl** | 11.4 ± 7.9 | > 4.4E+06 |
| **5·HCl** | 0.279 ± 0.154 | > 1.8E+08 |
| **6** | 0.169 ± 0.041 | > 3.0E+08 |
| **7·HCl** | 0.497 ± 0.055 | > 1.0E+08 |
| **8·HCl** | 0.845 ± 0.108 | > 5.9E+07 |
| **10** | 0.390 ± 0.012 | > 1.3E+08 |
| **12** | 4.836 ± 1.053 | > 1.0E+07 |
| **13·HCl** | 0.081 ± 0.043 | > 6.2E+08 |
| **14·HCl** | 0.088 ± 0.02 | > 5.7E+08 |
| **15** | 0.082 ± 0.007 | > 6.1E+08 |
| **16** | 0.067 ± 0.005 | > 7.5E+08 |

### 2.4 Results: in vitro activity of dual-targeting compounds against multi drug resistant PfDd2 line

Selected compounds **(1·HCl, 4·HCl, 8·HCl, 9, 13·HCl, 15, 19·HCl, 31)** were assessed for growth inhibitory activity against the multidrug-resistant *P*. *falciparum* line Dd2 (resistant to **CQ, PYR** and mefloquine). All the selected compounds were less potent on PfDd2, compared with Pf3D7 (Table III). However, the IC₅₀s of all compounds on PfDd2 were lower than 50 nM, except for **8** hydrochloride for which the IC₅₀ was 138nM.

**Table III. Comparative in vitro activity (IC₅₀) of selected compounds against drug-sensitive P. falciparum line 3D7 versus multidrug-resistant P. falciparum line Dd2. IC₅₀ values are presented as mean ± SD with n ≥ 2.**

| **Compound n°** | Pf3D7 IC50 (nM) | PfDd2 IC50 (nM) |
|---|---|---|
| **1** | 0.170 ± 0.018 | 19.8 ± 0.9 |
| **4** | 0.173 ± 0.122 | 6.2 ± 0.5 |
| **8** | 0.845 ± 0.108 | 137.9 ± 25.4 |
| **9** | 0.172 ± 0.027 | 16.9 ± 3.2 |
| **13** | 0.081 ± 0.043 | 11.1 ± 7.2 |
| **15** | 0.082 ± 0.007 | 4.0 ± 0.4 |
| **19** | 0.173 ± 0.021 | 41.5 ± 11.9 |
| **31** | 0.404 ± 0.136 | 22.8 ± 2.6 |

### 3 In vivo activity of selected dual-targeting compounds in a Plasmodium berghei experimental model of malaria

### 3.1 Materials and Methods

The oral efficacy of the three compounds at different concentrations (hydrochloride salts of compounds **1, 8, 13**) (Experiments 1, 2 and 3) as well as **compound 16** (Experiment 3) was examined using the Peters 4-day suppressive test.

### 3.1.1 Formulation of compounds

Dual-targeting compounds were formulated in 7% DMSO and 25% HPβCD in DPBS, pH 5.5 - 7 (Experiments 1 and 2) or in 6% DMSO and 25% HPβCD in DPBS, pH 5.5 - 7 (Experiment 3). Briefly, a 50% w/v solution of (2-hydroxypropyl)-β-cyclodextrin (Sigma-Aldrich cat # 332607) (HPβCD) was first prepared in distilled water on a slow-moving shaking table. Compounds powder was dissolved in DMSO and sonicated for 10 min. Appropriate volume of 50%W/v HPβCD was added dropwise under gentle swirling. After 10 min sonication, DPBS was added dropwise under gentle swirling and the mixture was sonicated again for 10 min. After checking the pH (5.5 - 7), the solution was filtered (0.22µm) and conserved at room temperature for max 2 days. Final concentration of compounds was 2.5 mg/mL, 1 mg/mL or 0.1 mg/mL for mice treatment with 25 mg/kg, 10 mg/kg or 1mg/kg doses. **CQ** was diluted in PBS.

### 3.1.2 P. berghei infection and compound administration

Protocols for experimental model of malaria and test of new anti-malarial compound have been approved by the local ethic committee (CEEA-75: Comité d'Ethique en Expérimentation Animale, Nord -Pas de Calais, France: APAFIS#18905-2019020111166978v2). 10 weeks-old Balb/C male mice were purchased from Janvier Labs.

*P. berghei* ANKA parasites stabilates conserved in liquid nitrogen were thawed and injected intraperitoneally (ip) into 2-3 mice (10⁶ infected red blood cells (RBC) / mouse). When parasitemia reached 3-5%, mice were bled and groups of six BALB/c male mice were infected with 10⁶ P. *berghei* ANKA parasites (day 0), and treated *per os (p.o.)* twice a day on days 0, 1, 2 and 3. In Experiments 1 and 3, three mice were given p.o. **CQ** at 10 mg/kg, as a positive control once a day, for four days. As negative control, six mice received 7% DMSO 25% HPβCD in DPBS (Experiments 1 and 2) or 6% DMSO 25% HPβCD in DPBS. On day 4 post infection (p.i.), the peripheral blood parasitemia of all mice was evaluated via microscopic examination of Giemsa-stained thin blood smears. Parasitemia was then followed for two weeks.

Three experiments were performed:
**Experiment 1:**
   6 Balb/C mice received **compound 1·HCl,** 2 doses **25 mg/kg** *p.o*. per day for 4 days
   6 Balb/C mice received **compound 1·HCl,** 2 doses **10 mg/kg** *p.o.* per day for 4 days
   6 Balb/C mice received DMSO (same formulation as compounds) *p.o*. twice a day for 4 days
   3 Balb/c mice received **CQ, 1** dose 10 mg/kg *p.o*. per day for 4 days
**Experiment 2:**
   6 Balb/C mice received **compound 8·HCl,** 2 doses **10 mg/kg** *p.o.* per day for 4 days
   6 Balb/C mice received **compound 13·HCl,** 2 doses **10 mg/kg** *p.o*. per day for 4 days
   6 Balb/C mice received DMSO (same formulation as compounds) *p.o*. twice a day for 4 days
**Experiment 3:**
   6 Balb/C mice received **compound 1·HCl,** 2 doses **1 mg/kg** *p.o*. per day for 4 days
   6 Balb/C mice received **compound 8·HCl,** 2 doses **1 mg/kg** *p.o.* per day for 4 days
   6 Balb/C mice received **compound 13·HCl,** 2 doses **1 mg/kg** *p.o.* per day for 4 days
   5 Balb/C mice received **compound 16**, 2 doses **1 mg/kg** *p.o*. per day for 4 days
   6 Balb/C mice received DMSO (same formulation as compounds) *p.o*. twice a day for 4 days
   3 Balb/c mice received **CQ,** 1 dose 10 mg/kg *p.o*. per day for 4 days

### 3.2 Results: in vivo activity of dual-targeting compounds against P. berghei

Results show that following treatment at 10mg/kg, parasites were undetectable at day 4 p.i. in the **compound 1-, compound 8-** or **compound 13**-treated mice whereas the vehicle control group showed a parasitemia of -1.2% and -2.5% in experiment 1 and 2, respectively (Fig. 1; Fig. 2). The follow-up of parasitemia showed that while parasitemia continue increasing in the vehicle control group (7.7% and 10.8% at day 6 p.i. in Experiment 1 and 2, respectively), parasites remained undetectable in **compound 1-, compound 8-** or **compound 13**-treated mice until the end of the experiments.

When mice were treated at 1mg/kg with **compound 1, compound 8, compound 13** or **compound 16,** at day 4 p.i.: parasites were detected in 2/6 **compound 13**-treated mice (mean parasitemia = 0.12%, n = 6) and in 1/5 **compound 16**-treated mice (mean parasitemia = 0.02%, n = 5) (Fig. 3); all vehicle-treated mice showed blood parasites (mean parasitemia = 2.41%, n = 6); and parasites were undetectable in **1-** or **compound 8-**treated mice.

At day 6 p.i., parasites were detected in 1/6 **compound 1**-treated mice, in 2/6 **compound 8-**treated mice, in 4/6 **compound 13**-treated mice and in 3/5 **compound 16**-treated mice (see Table below). At the end of the experiment (day 10 p.i.), parasites were detected in 1/6 **compound 1**-treated mice, in 4/6 **compound 8**-treated mice, in 5/5 **compound 13**-treated mice and in 5/5 **compound 16**-treated mice.

These results show that **compound 11** and **compound 8** are the most active compounds against P. *berghei* infection.

**Table IV. The table above shows the follow-up of parasitemia (mean ± sd) as well as the number of mice showing blood parasites on days 4, 5, 6, 7, 8 and 10 post infection (p.i.).**

| | **day 4 p.i.** | **day 5 p.i.** | **day 6 p.i.** | **day 7 p.i.** | **day 8 p.i.** | **day 10 p.i.** |
|---|---|---|---|---|---|---|
| **DMSO** | 2,41 ± 1,88 (6/6) | 6,08 ± 1,98 (6/6) | 9,00 ± 3,35 (6/6) | 9,6 ± 2,18 (6/6) | 8,40 ± 3,45 (3/3) | 11,05 ± 4,48 (2/2) |
| **1.HCl** | 0 (0/6) | 0,07 ± 0,16 (1/6) | 0,15 ± 0,37 (1/6) | 0,31 ± 0,76 (1/6) | 1,31 ± 3,21 (1/6) | 0,7 ± 1,72 (1/6) |
| **8.HCl** | 0 (0/6) | 0 (0/6) | 0,08 ± 0,13 (2/6) | 0,32 ± 0,49 (2/6) | 1,37 ± 2,07 (3/6) | 2,41 ± 2,84 (4/6) |
| **13.HCl** | 0,12 ± 0,18 (2/6) | 0,55 ± 0,83 (3/6) | 2,05 ± 2,37 (4/6) | 2,39 ± 2,22 (5/6) | 3,94 ± 3,06 (5/6) | 6,02 ± 2,66 (5/5) |
| **16** | 0,02 ± 0,04 (1/5) | 0,48 ± 0,72 (2/5) | 2,22 ± 2,44 (3/5) | 5,21 ± 7,86 (3/5) | 5,33 ± 5,64 (5/5) | 9,25 ± 7,38 (5/5) |
| **CQ** | 0 (0/3) | 0 (0/3) | 0 (0/3) | 0 (0/3) | 0 (0/3) | 0 (0/3) |

### 4 Conclusion

The compounds of the invention are highly potent against P. *falciparum in vitro* sensitive and multi-resistant strains and they exhibit very high selectivity indexes to HEK293 cells.

In addition, the DHFR inhibitor moiety still retained its capacity to inhibit *in vitro* the enzymatic activity of mutated DHFR. 12 compounds have been tested in cytotoxicity assays against HEK293 cells and showed very high Selectivity Indexes. Importantly, 8 compounds were also very potent against *P*. *falciparum* multidrug resistant Dd2 strain *in vitro.* In an *in vivo* mouse malaria model, three compounds (**1, 8, 13**) administered by oral route (at 10 mg/kg) achieved parasite clearance after oral administration. Moreover, two of them are extremely potent as they are still active according to the Peters test when administered at 1mg/kg.

## Claims

1. A compound of formula (I) below: 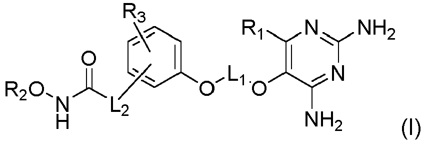 wherein
∘ L₁ is a (C₁-C₆) alkylene,
∘ R₁ is H, a (C₁-C₆)alkyl group or a (C₃-C₆)cycloalkyl group,
∘ R₂ is H, a (C₁-C₆)alkyl group, or a (C₁-C₆)alkylaryl group, said alkylaryl being optionally substituted by -OH, -OR^{a} or -OC(O)R^{b}, with R^{a} and R^{b} independently representing a (C₁-C₆)alkyl group,
∘ R₃ is H, a (C₁-C₆)alkyl group, a halogen, a (C₁-C₆)alkoxy group, -OH, -NHR^{c}, (C₁-C₆)haloalkyl group, -CONHR^{d}, -NHCOR^{e}, with R^{c} and R^{d} independently representing H or a (C₁-C₆)alkyl group, and R^{e} representing a (C₁-C₆)alkyl group,
∘ L₂ is a bond, a (C₁-C₁₂) alkylene, a (C₂-C₁₂) alkenylene, -NH-(C₁-C₁₂) alkylene, -NH-(C₂-C₁₂) alkenylene, -CO-NH- (C₁-C₁₂) alkylene, -CO-NH- (C₂-C₁₂) alkenylene, -O-(C₁-C₁₂) alkylene, -NH-CO-(C₁-C₁₂) alkylene or (C₁-C₁₂) alkylene-O-arylène-(C₂-C₁₂) alkenylene,
or a pharmaceutically acceptable salt or solvate thereof.

2. The compound of claim 1, wherein
∘ L₁ is a (C₂-C₄) alkylene,
∘ R₁ is H, a (C₁-C₄)alkyl group or a (C₃-C₄)cycloalkyl group,
∘ R₂ is H or a (C₁-C₄)alkylphenyl group, said phenyl being optionally substituted on the aryl moiety by -OR^{a} or -OC(O)R^{b}, with R^{a} and R^{b} independently representing a (C₁-C₄)alkyl group,
∘ R₃ is H, a (C₁-C₄)alkyl group, a halogen, a (C₁-C₄)alkoxy group, -OH, -NH₂, -CF₃, - CONH₂, -NHCOCH₃,
∘ L₂ is a bond, a (C₁-C₁₂) alkylene or a (C₂-C₁₂) alkenylene, -CO-NH- (C₁-C₁₂) alkylene, - CO-NH- (C₂-C₁₂) alkenylene, -NH-CO-(C₁-C₆) alkylene or (C₁-C₁₂) alkylene-O-arylene-(C₂-C₁₂) alkenylene,
or a pharmaceutically acceptable salt or solvate thereof.

3. The compound of claim 1 or 2, wherein L₁ is -CH₂-CH₂- or -CH₂-CH₂-CH₂-, preferably -CH₂-CH₂-CH₂-.

4. The compound of any of claims 1 to 3, wherein R₁ is H or a C₁-C₃ alkyl or a cyclopropryl group, preferably ethyl.

5. The compound of any of claims 1 to 4, wherein R₂ is H, benzyl or 4-AcO-Ph-CH₂-, preferably H.

6. The compound of any of claims 1 to 4, wherein R₃ is H, halogen or a methoxy group.

7. The compound of any of claims 1 to 6, wherein L₂ is a bond, -CH₂-CH₂- or -CH=CH-,-CH₂-O-Ph-CH=CH-, -CO-NH-(CH₂)₆-, -NH-CO-(CH₂)₆-, -NH-CO-(CH₂)₄-, preferably a bond, -CH₂-CH₂- or -CH=CH-.

8. The compound of any of claims 1 to 7, wherein it is in the form of an HCl addition salt.

9. The compound of claim 1, wherein the compound is selected from the group consisting of:
| | |
|---|---|
| **1** | 3-{2-[3-(2,4-Diamino-6-ethylpyrimidin-5-yloxy)propoxy]phenyl}-*N*-hydroxypropanamide |
| **2** | 3-{2-[2-(2,4-Diamino-6-ethylpyrimidin-5-yloxy)ethoxy]phenyl}-*N*-hydroxypropanamide |
| **3** | 3-{2-[4-(2,4-Diamino-6-ethylpyrimidin-5-yloxy)butoxy]phenyl}-*N*-hydroxypropanamide |
| **4** | 3-{2-[3-(2,4-Diamino-6-ethylpyrimidin-5-yloxy)propoxy]-4-methoxyphenyl}-*N-*hydroxypropanamide |
| **5** | 3-{4-Chloro-2-[3-(2,4-diamino-6-ethylpyrimidin-5-yloxy)propoxy]phenyl}-*N*-hydroxypropanamide |
| **6** | *N*-(4-Acetoxybenzyloxy)-3-(2-(3-(2,4-diamino-6-ethylpyrimidin-5-yloxy)propoxy)phenyl)propanamide |
| **7** | *N*-(Benzyloxy)-3-(2-(3-(2,4-diamino-6-ethylpyrimidin-5-yloxy)propoxy)phenyl)propanamide |
| **8** | 3-[3-(2,4-Diamino-6-ethylpyrimidin-5-yloxy)propoxy]-*N*-hydroxybenzamide |
| **9** | 4-Chloro-3-[3-(2,4-diamino-6-ethylpyrimidin-5-yloxy)propoxy]-*N*-hydroxybenzamide |
| **10** | 3-(3-[2,4-Diamino-6-ethylpyrimidin-5-yloxy)propoxy]-4-fluoro-*N*-hydroxybenzamide |
| **11** | *N*-(4-Acetoxybenzyloxy)-3-[3-(2,4-diamino-6-ethylpyrimidin-5-yloxy)propoxy]benzamide |
| **12** | *N*-(Benzyloxy)-3-[3-(2,4-diamino-6-ethylpyrimidin-5-yloxy)propoxy]benzamide |
| **13** | 3-{2-[3-(2,4-Diamino-6-ethylpyrimidin-5-yloxy)propoxy]phenyl}-*N*-hydroxyacrylamide |
| **14** | 3-{2-[3-(2,4-Diamino-6-ethylpyrimidin-5-yloxy)propoxy]-4-methoxyphenyl}-*N*-hydroxyacrylamide |
| **15** | 3-{4-Chloro-2-[3-(2,4-diamino-6-ethylpyrimidin-5-yloxy)propoxy]phenyll-*N*-hydroxyacrylamide |
| **16** | *N*-(4-Acetoxybenzyloxy)-3-{2-[3-(2,4-diamino-6-ethylpyrimidin-5-yloxy)propoxy]phenyl}acrylamide |
| **17** | *N*-(Benzyloxy)-3-{2-[3-(2,4-diamino-6-ethylpyrimidin-5-yloxy)propoxy]phenyl}acrylamide |
| **18** | 3-{3-[2-(2,4-Diamino-6-ethylpyrimidin-5-yloxy)ethoxy]phenyl}-*N*-hydroxyacrylamide |
| **19** | 3-{3-[3-(2,4-Diamino-6-ethylpyrimidin-5-yloxy)propoxy]phenyl}-*N*-hydroxyacrylamide |
| **20** | 3-{4-[3-(2,4-Diamino-6-ethylpyrimidin-5-yloxy)propoxy]phenyl}-*N*-hydroxyacrylamide |
| **21** | 3-(3-{3-[3-(2,4-Diamino-6-ethylpyrimidin-5-yloxy)propoxy]benzyloxy}phenyl)-*N-*hydroxyacrylamide |
| **22** | 4-(2-{3-[(2,4-Diamino-6-ethylpyrimidin-5-yl)oxy]propoxy}phenyl)-*N*-hydroxybutanamide |
| **23** | 5-(2-{3-[(2,4-Diamino-6-ethylpyrimidin-5-yl)oxy]propoxy}phenyl)-*N*-hydroxypentanamide |
| **24** | 3-(2-{3-[(2,4-Diamino-6-cyclopropylpyrimidin-5-yl)oxy]propoxy}phenyl)-*N*-hydroxypropanamide |
| **25** | 3-(3-{3-[(2,4-Diamino-6-ethylpyrimidin-5-yl)oxy]propoxy}phenyl)-*N*-hydroxypropanamide |
| **26** | 3-(4-{3-[(2,4-Diamino-6-ethylpyrimidin-5-yl)oxy]propoxy}phenyl)-*N*-hydroxypropanamide |
| **27** | 4-{3-[(2,4-Diamino-6-ethylpyrimidin-5-yl)oxy]propoxy}-*N*-hydroxybenzamide |
| **28** | *N*¹-(2-{3-[(2,4-diamino-6-ethylpyrimidin-5-yl)oxy]propoxy}phenyl)-*N*⁶-hydroxyadipamide |
| **29** | *N*¹-(2-(3-((2,4-diamino-6-ethylpyrimidin-5-yl)oxy)propoxy)phenyl)-*N*⁸-hydroxyoctanediamide |
| **30** | 3-{3-[(2,4-Diamino-6-ethylpyrimidin-5-yl)oxy]propoxy}-*N*-[7-(hydroxyamino)-7-oxoheptyl]benzamide, and |
| **31** | 4-{3-[(2,4-Diamino-6-ethylpyrimidin-5-yl)oxy]propoxyl-*N*-[7-(hydroxyamino)-7-oxoheptyl]benzamide |
or a pharmaceutically acceptable salt or solvate thereof.

10. A pharmaceutical or veterinary composition comprising a compound of formula (I) as defined in any of claims 1 to 9, and a pharmaceutically or veterinary acceptable carrier.

11. The pharmaceutical or veterinary composition of claim 10, wherein the composition further comprises another therapeutic compound, preferably an antiparasitic agent and/or an antibiotic compound.

12. A compound of any of claims 1 to 9 or a composition of claim 10 or 11 for use as drug.

13. A kit comprising:
- a composition of claim 10 or 11;
- a second composition comprising at least another therapeutic compound, preferably an antiparasitic agent and/or an antibiotics, and
- preferably instructions for using said kit,
as a combination product for simultaneous, separate and staggered use as drug.

14. The compound or the composition for use of claim 12 or the kit for use of claim 13, for preventing or treating a parasitic disease, such as malaria.

15. A method for preparing the compounds of formula (I) as defined in any of claims 1 to 9, said method comprising the following steps:
a) Subjecting a compound of formula (II) below: 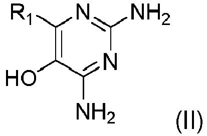
with R₁ as defined in any of claims 1 to 9,
to a Williamson ether synthesis with a compound of formula (III) below: 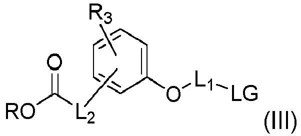
with R₃, L₁ and L₂ as defined in any of claims 1 to 9,
R representing a (C₁-C₄)alkyl group, preferably a methyl or ethyl group, more preferably a methyl group, and
LG representing a leaving group,
to obtain a compound of formula (IV) below 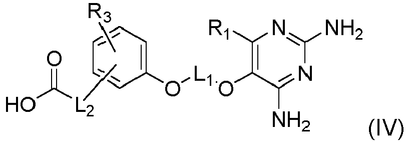
with R₁, R₃,L₁ and L₂ as defined above, and
b) Subjecting the compound of formula (IV) as obtained in step a) to a peptidic coupling with a compound formula (V) below:
H₂N-O-PG (V)
PG being a THP protecting group or a (C₁-C₆)alkyl group, or a (C₁-C₆)alkylaryl group, said alkylaryl being optionally substituted by -OH, -OR^{a}, -OC(O)R^{b}, with R^{a} and R^{b} independently representing a (C₁-C₆)alkyl group,
or an acid addition salt of the compound formula (V), such as an hydrochloride salt thereof.
